# EUROPEAN PATENT SPECIFICATION

(11) **EP 1 898 809 B1**
(45) Date of publication and mention of the grant of the patent: **26.08.2015**
(21) Application number: 06784715.2
(22) Date of filing: 09.06.2006
(51) Int. Cl.: A61B 17/28, A61B 17/00

(54) **SURGICAL INSTRUMENT**
CHIRURGISCHES INSTRUMENT
INSTRUMENT CHIRURGICAL

(30) Priority: 16.06.2005 US 690968 P; 25.08.2005 US 711347 P; 07.02.2006 US 349757; 07.02.2006 US 349769; 07.02.2006 US 350292; 17.03.2006 US 378457
(43) Date of publication of application: 19.03.2008
(73) Proprietor: Marsh Surgical, Inc., Glencoe, IL 60022 (US)
(72) Inventor: PERLIN, Alfred, Highland Park, Illinois 60035 (US)
(74) Representative: Lambrinos, Matthew Franklin
(86) International application number: PCT/US2006/022548
(87) International publication number: WO 2006/138175

(56) References cited:
- WO-A-96/04856
- DE-A1- 19 632 135
- DE-U1- 9 117 265
- DE-U1- 9 401 042
- US-A- 2 113 246
- US-A- 4 084 594
- US-A- 5 556 416
- US-A- 5 984 939
- US-A1- 2001 027 312

## Description

### FIELD OF THE INVENTION

The present invention relates generally to surgical instruments and, more particularly, to a surgical instrument having a detachable or separable sleeve assembly.

### BACKGROUND OF THE INVENTION

Laparoscopic instruments are used during laparoscopy procedures, which are generally used to examine a patient and/or to perform minor surgery on the patient. For example, a laparoscopic instrument can be used to examine the patient's abdominal cavity for signs of disease or abnormality. In addition, fully invasive surgery may be avoided by using the laparoscopic instrument to perform relatively minor surgery. Similarly, in minimally invasive arthroscopic procedures, such as on a knee joint, an arthroscopic instrument is used to access joints or bones.

The laparoscopic (or arthroscopic) instrument generally includes a grasping end and an operating end that are connected by a flexible hollow cylindrical shaft. The laparoscopic instrument is introduced into the patient through a cannula/trocar unit. After the laparoscopic instrument is inserted into the patient through a cannula that is anchored to the body via a small incision, the surgeon may insert one of a plurality of laparoscopic tools into the laparoscopic instrument to perform a particular surgical procedure. For example, if a grasping procedure is required the surgeon will insert a grasping tool in the laparoscopic instrument. Similarly, if a cutting procedure is required the surgeon will insert a cutting tool in the laparoscopic instrument.

One problem associated with current laparoscopic instruments is that they may cause the surgeon to lose his or her "feel" when changing laparoscopic tools. During surgery, the surgeon develops a particular "feel" associated with the location and positioning of the laparoscopic instrument relative to the patient's internal cavities. Because the surgeon may be required to perform several procedures during a single surgery, each procedure requiring a different laparoscopic tool, the surgeon may lose the "feel" when changing the laparoscopic tools.

In one exemplary scenario, the surgeon uses an examination tool to find the best location for performing a cutting procedure. After finding the best location, the surgeon retrieves the laparoscopic instrument from within the patient, replaces the examination tool with a cutting tool, and reinserts the laparoscopic instrument inside the patient. It can be time consuming and frustrating for the surgeon to locate, for a second time, the best location for performing the cutting procedure.

Another problem associated with current laparoscopic instruments is that they have a fixed grasping end and, therefore, limit the ability and/or comfort of the surgeon in attaining desired positions within the patient's body. Depending on the surgical procedure, the surgeon must often change the position of the laparoscopic instrument or contort his or her body position to reach various parts of a patient's internal cavity. For example, the surgeon will often attempt to achieve the best cutting position before performing a delicate cutting procedure by rotating and/or moving the grasping end of the laparoscopic tool at various uncomfortable and awkward positions. Because the grasping end of the laparoscopic instrument is fixed, the surgeon must perform the cutting procedure by grasping the laparoscopic tool at an uncomfortable or awkward position that decreases the likelihood of a successful surgical procedure, or must contort his or her body to access a hard-to-reach area of the patient's internal cavity.

Yet another problem associated with current laparoscopic instruments is that the surgeon must clasp the operating end together in order to hold a grasping tool in a closed position. Prolonged clasping results in hand fatigue and also undesirably ties up one of the surgeon's hands to perform other tasks. If the surgeon removes or relaxes his hand from the grasping end, then the grasping tool may lose its grip on the internal body structure it was grasping.

Thus, there is a need to provide a laparoscopic tool that allows the surgeon to retain the "feel" developed during a surgical procedure by changing laparoscopic tools without having to remove the laparoscopic instrument from within the patient's body. There is also a need for an adjustable grasping end for a laparoscopic or arthroscopic instrument for attaining desired and/or comfortable operating positions. There is yet another need for a laparoscopic or arthroscopic instrument that can lock a grasping tool in a fixed position without requiring manual clasping by the surgeon. The present invention fulfills these and other needs. The two part of claim 1 is based on US-A-5904939.

### SUMMARY OF THE INVENTION

The invention is disclosed in claim 1 and preferred embodiments are disclosed in the dependent claims. In an aspect not of the present invention, a method is presented for replacing a tool of a laparoscopic instrument without removing the laparoscopic instrument from a body. The method includes rotating a housing portion of the laparoscopic instrument to expose an end of the tool to be removed, removing the tool from the laparoscopic instrument without removing the laparoscopic instrument from the body, and inserting a second tool into a sleeve of the laparoscopic instrument. The method may further include registering the exposed end of the tool in at least one of a ball-receiving slot and a ball-receiving hole of the housing portion when the housing assembly portion is in a locked position or pressing a locking lever to unlock the housing assembly from a locked position. The rotating may include rotating the housing portion about an axis of the laparoscopic instrument selected from a group consisting of an X-axis, a Y-axis, and a Z-axis. The X-axis is any axis lying in the 3-dimensional space occupied by the laparoscopic instrument. Alternately, the rotating may include rotating the housing assembly about a hinge of the laparoscopic instrument, the housing assembly being pivotably coupled to the hinge via a hinge pin. The method may further include grasping a pair of handles when rotating the housing assembly. The pair of handles is attached to the housing assembly. The method may still further include linearly displacing the tool when the housing assembly is in a locked position to manipulate a tool device located at an opposing end of the tool.

In another aspect not of the present invention, a laparoscopic instrument includes a housing assembly that includes a pair of handles and a hinge portion. The hinge portion is pivotally connected to the housing portion and includes a tool knob extension for insertion into an incision of a body. The knob extension includes an open end and an elongated hollow shaft (sleeve) for receiving a first tool for insertion into the sleeve through the open end. The hinge portion is pivotable between a closed position and an open position and is positioned in the open position with the knob extension remaining in the body when changing the first tool with a second tool. The housing assembly may include a drum assembly having at least two drums connected to respective ones of the pair of handles. The drums rotate independently of one another to permit movement of the handles relative to one another. The drums also rotate together in a fixed relationship about an axis passing through the center of the drums to permit rotation of the handles in a fixed relationship about the axis. The hinge in the closed position may rotate around an axis perpendicular to an axis of the knob extension to achieve the open position. Alternately, the hinge portion in the closed position may rotate around an axis parallel to an axis of the knob extension to achieve the open position. The hinge portion may further include a locking lever for locking the hinge portion in at least the locked position and the open position. The housing assembly may include a ball-receiving slot for receiving a ball end of any of the first tool and the second tool when the hinge portion is in the closed position.

In yet another aspect not of the present invention, a method of replacing a tool of a laparoscopic instrument includes inserting a laparoscopic instrument into an incision of a body having a first tool and a pair of handles coupled to the first tool. A housing assembly of the laparoscopic instrument is rotated from a locked position to an open position to expose an end of the first tool. The first tool is removed and a second tool is inserted without removing the laparoscopic instrument from the body. The housing assembly is rotated from the open position to the locked position to couple the second tool to the handles of the laparoscopic instrument. The method may further pressing a locking lever to release the housing assembly from the locked position or pressing the locking lever to release the housing assembly from the open position. The method may further rotating one of the handles to cause a linear movement of a tool device of the first tool or the second tool. The method may still further include rotating the housing assembly about any axis of the laparoscopic instrument lying in the 3-dimensional space occupied by the laparoscopic instrument.

In still another aspect not of the present invention, a method of exchanging tools in a surgical instrument includes inserting a first tool through an elongated sleeve of the surgical instrument, moving part of the surgical instrument to permit removal of the first tool from the elongated sleeve, removing the first tool from the elongated sleeve, and inserting a second tool into the elongated sleeve. The surgical instrument can be a laparoscopic instrument or an arthroscopic instrument. The method may further include inserting the surgical instrument together with its elongated sleeve through a cannula into the body. The moving may include rotating a housing assembly of the surgical instrument to expose an end of the first tool to be removed.

In an aspect of the present invention, a laparoscopic instrument includes a first handle pivotally rotatable among at least two lockable positions, or, in another aspect of the present invention, among at least three lockable positions. The instrument may further include a drum attached to the first handle and a shaft passing through the drum. The drum is rotatable around the shaft to cause the first handle to the pivotally rotated among the at least two lockable positions. The instrument includes a second handle pivotally rotatable among the at least two or three lockable positions with the first handle. The instrument may further include a second drum attached to the second handle. The shaft also passes through the second drum, which is rotated around the shaft to cause the second handle to be pivotally rotated among the at least two or three lockable positions. The instrument may further include a drum attached to the first handle, a second handled attached to a second drum, a tool drum coupled to the drum and the second drum, and a shaft passing through all three drums so as to be rotatable among the at least two or three lockable positions. The shaft may further include winglets disposed along a length of the shaft and positioned to lock together any combination pair of the drum, the second drum, and the tool drum while the shaft is rotated among the at least two or three lockable positions. The instrument may still further include a housing, a drum attached to the handle, a shaft passing through the drum for securing the drum along a central axis of the housing and a push-button connected to the shaft for moving the shaft between one of the at least two lockable positions and another position. The instrument may further include a tool drum connected to the handle and a tool having a ball end and a tool end. The ball end of the tool is removably connected to the tool drum in any of the at least two or three lockable positions.

In still another aspect of the present invention, a laparoscopic instrument has a transverse axis along which a tip is disposed. The laparoscopic instrument includes a trigger handle pivotally rotatable among at least two lockable positions about an axis orthogonal to the transverse axis. The instrument may further include a trigger drum attached to the trigger handle, a tool drum rotationally secured to the trigger drum in the at least two lockable positions, and a fixing drum attached to a fixing handle. The fixing drum is rotationally secured to the trigger drum when the trigger drum is in an unlocked position. The instrument further includes a winged shaft for axially securing the trigger drum, the tool drum, and the fixing drum to each other. The winged shaft can include one or more winglets, spline parts, keys, or pins. The winged shaft is movable between a first position and a second position. The tool drum and the trigger drum are rotationally secured with respect to each other in the first position, and the trigger drum and the fixing drum are rotationally secured with respect to each other in the second position. The first position corresponds to one of the at least two lockable positions.

In still yet another aspect not of the present invention, a method of using a laparoscopic instrument has a trigger drum connected to a handle of the instrument. The method includes rotating the trigger drum from a first drum locked position to a second drum locked position. The method may further include unlocking the trigger drum from the first drum locked position before rotating the drum to the second drum locked position. The rotating may include rotating the trigger drum together with a fixing drum to a second drum position. The method may further include depressing a push-button to unlock the trigger drum from the first drum locked position and releasing the push-button to lock the trigger drum and the fixing drum in the second drum locked position. The method may further include disengaging the fixing drum from at least one push pin when depressing the push-button. The method may further include positioning a shaft in a first shaft position to rotationally secure the trigger drum to the tool drum in the first drum locked position, urging the shaft from the first shaft position to a second shaft position to rotationally secure the trigger drum to the fixing drum in an unlocked drum position, the trigger drum being rotationally released from the tool drum in the unlocked drum position, rotating the fixing drum together with the trigger drum to the second drum position, and urging the shaft from the second shaft position to the first shaft position to rotationally secure the trigger drum to the tool drum in the second drum locked position. The method may further include inserting a tool into the laparoscopic instrument and linearly displacing the tool to manipulate a tool device located at an end of the tool. The linearly displacement may be carried out by rotating a handle that is coupled to the trigger drum.

According to another aspect not of the present invention, a laparoscopic instrument includes a first handle and a second handle. The first handle includes a latching mechanism. The second handle is pivotally coupled to the first handle and includes a locking part. The first handle and the second handle are in a locked position when the latching mechanism is engaged with the locking part, and the first handle and the second handle are in an open position when the latching mechanism is disengaged from the locking part. The latching mechanism may include at least one latching tooth and the locking part includes at least one locking tooth, the latching tooth being engaged to the locking tooth in the locked position. The latching mechanism may further include a latching lever pivotably coupled to the first handle. The latching lever is aligned with the first handle in the locked position. A free end of the latching lever can be pivotable in a direction away from the first handle to achieve the open position. The latching mechanism may include a lever limiter positioned along the rotational path of the latching lever for limiting the rotation of the latching lever. The lever limiter can be coupled proximate a free end of the latching lever. The latching mechanism may include a latching teeth-engaging part attached to a free end of a latching lever and the locking part includes a locking teeth-engaging part. The latching lever is pivotally coupled to the first handle at a fixed end, and the latching lever is aligned with the first handle in the locked position. The latching teeth-engaging part is aligned and engaged to the locking teeth-engaging part when the first handle is in the locked position.

In another aspect not of the present invention, a method is directed to locking and unlocking a first handle of a laparoscopic instrument relative to a second handle. The method includes urging the first handle towards the second handle to engage a latching mechanism of the first handle to a locking part of the second handle. The method may include biasing the latching mechanism against the locking part to secure a plurality of teeth to each other. At least one of the plurality of teeth may be located on the latching mechanism and a corresponding one of the plurality of teeth may be located on the locking part. The method may further include pivoting the latching mechanism away from the first handle to unlock the first handle from the second handle. The method may still further include mounting the latching mechanism on a latching lever pivotally coupled to the first handle and rotating a free end of the latching lever to disengage the latching mechanism from the locking part. The method may further include limiting the pivoting motion of the latching lever using a lever limiter. The lever limited may be coupled to the first handle proximate the free end of the latching lever. The method may also include rotating the free end of the latching lever in a direction that is substantially perpendicular to the pivoting direction of the first handle towards the second handle. Still further, the method may include coupling the latching mechanism to a grasping end of the first handle.

In still another aspect not of the present invention, a laparoscopic instrument includes a pair of handles that are pivotable between a locked position and an open position. The laparoscopic instrument further includes a latching lever, a lever limited, a tooth-engaging part, and a locking part. The latching lever is pivotally coupled to a trigger handle of the pair of handles at a pivoting point. The lever limiter is coupled to the trigger handle near a free end of the latching lever and is directed to restricting the pivoting motion of the latching lever. The tooth-engaging part is coupled to the trigger handle. The locking part is coupled to a fixing handle of the pair of handles such that the locking part is engaged to the tooth-engaging part when the pair of handles is in the locked position, and such that the locking part is disengaged from the tooth-engaging part when the pair of handles is in the open position. The locking part may be biased towards the tooth-engaging part via a frictional force exerted by at least one pair of interlocking teeth.

In another aspect not of the present invention, a method of replacing a tool from a sleeve of a surgical instrument without having to remove the sleeve from a body is provided. The surgical instrument may be of the laparoscopic or arthroscopic type. The method includes urging a releasable locking mechanism of the surgical instrument from a locked position to an unlocked position, thereby causing the sleeve to be decoupled from the surgical instrument, and withdrawing at least part of the surgical instrument from the sleeve such that the tool exits the sleeve together with the at least part of the surgical instrument. The method may further include rotating a housing assembly of the surgical instrument to expose an end of the tool, and separating the tool from the laparoscopic instrument. A second tool is inserted through the sleeve. The urging can include depressing a locking structure releasably interlocked with a portion of the tool or moving a locking plate in a direction generally away from the tool.

According to still another aspect not of the present invention, there is provided a surgical instrument including a housing assembly releasably coupled to an elongated hollow sleeve via a locking mechanism movable between at least a locked position and an unlocked position such that in the locked position the sleeve is secured to the housing assembly and in the unlocked position the sleeve is separable from the housing assembly. The locking mechanism may include a movable locking plate and the housing assembly includes an elongated shaft having a groove formed along its length, the movable locking plate being shaped to fit within at least part of the groove when the locking mechanism is in the locked position. The surgical instrument may further include a rotatable knob coupled to the locking plate and secured to the elongated hollow shaft, and a tool inserted through the elongated hollow shaft. The surgical instrument is of the laparoscopic or arthroscopic type, for example. The sleeve may include an insulating layer. The housing assembly may include a pair of handles, and the surgical instrument may further include a hinge portion pivotally connected to the housing assembly and including a sleeve for insertion into an incision of a body, the sleeve having an open end and an elongated hollow shaft for receiving a first tool for insertion into the hollow shaft through the open end, the hinge portion being pivotable between a closed position and an open position, the hinge portion being positioned in the open position with the sleeve remaining in the body when changing the first tool with a second tool. The hinge portion of the surgical instrument may further include a locking lever for locking the hinge portion in at least the locked position and the open position. The housing assembly may further include a ball-receiving slot for receiving a ball end of a tool when the hinge portion is in the closed position.

According to yet another aspect of the present invention, a surgical instrument includes a pair of handles, a drum assembly coupled to the pair of handles, a rotatable knob assembly including a locking mechanism, an elongated hollow sleeve coupled to the knob, and a tool inserted through at least the knob and the elongated hollow sleeve, wherein the locking mechanism releasably interconnects the knob assembly with the drum assembly such that in a locked position the knob assembly is secured to the drum assembly and in an unlocked position the knob assembly is separable from the drum assembly. The drum assembly may include at least two drums connected to respective ones of the pair of handles, the drums rotating independently of one another to permit movement of the handles relative to one another, the drums also rotating together in a fixed relationship about an axis passing through the center of the drums to permit rotation of the handles in a fixed relationship about the axis. The rotatable knob assembly may include a knob having an aperture and a bearing shaft disposed into the aperture and having a groove formed thereon for receiving a portion of the locking mechanism in the locked position. The drum assembly may include a receiving slot for receiving an end of the tool when the locking mechanism is in the locked position. The surgical instrument may further include a hinge portion pivotally connected to the drum assembly, the hinge portion being pivotable between a closed position and an open position, an end of the tool being exposed when the hinge portion is in the open position.

The above summary of the present invention is not intended to represent each embodiment, or every aspect, of the present invention. Additional features and benefits of the present invention are apparent from the detailed description, figures, and claims set forth below.

### BRIEF DESCRIPTION OF THE FIGURES

FIG. 1A is an exploded perspective view showing a first portion of a laparoscopic instrument according to one embodiment of the present invention.
FIG. 1AI is a perspective bottom view of the tool drum shown in FIG. 1A according to an embodiment of the present invention.
FIG. 1B is an exploded perspective view showing a second portion to the laparoscopic instrument shown in FIG. 1A.
FIG. 2 is an assembly perspective view of the laparoscopic instrument of FIGs. 1A and 1B.
FIG. 3 is a front view of the laparoscopic instrument of FIGs. 1A and 1B.
FIG. 4 is a top view of the laparoscopic instrument of FIGs. 1A and 1B.
FIG. 5 is a back view of the laparoscopic instrument of FIGs. 1A and 1B.
FIG. 6 is a bottom view of the laparoscopic instrument of FIGs. 1A and 1B.
FIG. 7 is a left-side view of the laparoscopic instrument of FIGs. 1A and 1B, showing a tool end of the laparoscopic instrument.
FIG. 8 is a right-side view of the laparoscopic instrument of FIGs. 1A and 1B, showing a handle-end of the laparoscopic instrument.
FIG. 9 is an exploded perspective view showing a drum subassembly of the laparoscopic instrument of FIGs. 1A and 1B.
FIG. 10 is an assembly perspective view showing interior details of the drum subassembly of FIG. 9.
FIG. 11 is a planar cross-sectional view of the drum subassembly of FIG. 9.
FIGs. 12A-12C are perspective views of an assembly comprising a winged shaft and a handle drum according to three alternative embodiments of the present invention, respectively.
FIG. 13 is a cross-sectional view representing the interaction between a winged shaft and a drum subassembly according to an embodiment of the present invention.
FIG. 14 is a perspective cross-sectional view of the drum subassembly of FIG. 9 showing a push-button in a fully depressed position and a pair of handles in a first position.
FIG. 15 is a perspective cross-sectional view showing the push-button of FIG. 14 in the fully depressed position and the pair of handles in a second position.
FIG. 16 is a perspective cross-sectional view showing the push-button of FIG. 14 in an un-depressed position and the pair of handles in the second position.
FIG. 17 is a partial exploded perspective view showing a shotgun subassembly of the laparoscopic instrument of FIGs. 1A and 1B in an open breech position.
FIG. 18 is a perspective view of the shotgun subassembly of FIG. 17 in an open breech position exposing an insertion end of a laparoscopic tool.
FIG. 19 is a perspective view of the shotgun subassembly of FIG. 17 showing an assembled shotgun subassembly in an open breech position.
FIG. 20A is a representative diagrammatic front view showing an alternative embodiment of a shotgun subassembly rotating about an X-axis of a laparoscopic instrument.
FIG. 20B is a representative side view of FIG. 20A.
FIG. 21A is a representative top view showing another alternative embodiment of a shotgun subassembly rotating about a Y-axis of a laparoscopic instrument.
FIG. 21B is a representative front view of FIG. 22A.
FIG. 22A is a perspective view of a pair of handles of the laparoscopic instrument of FIGs. 1A and 1B in an open aligned position.
FIG. 22B is a perspective view of the pair of handles of FIG. 22A in a locked position.
FIG. 23A is a perspective view of the pair of handles of FIG. 22A in a closed offset position.
FIG. 23B is a perspective view of the pair of handles of FIG. 22A in an open offset position.
FIG. 24 is a perspective view of a surgical instrument illustrating an arrangement for removing the surgical instrument from a sleeve that remains in situ, according to another embodiment of the present invention.
FIG. 25 is a side view of a surgical instrument that is partially retracted from an insulated sleeve and a cut-away view of the insulated sleeve that is inserted into a body through a cannula/trocar unit anchored through an incision.

While the invention is susceptible to various modifications and alternative forms, specific embodiments are shown by way of example in the drawings and are described in detail herein. It should be understood, however, that the invention is not intended to be limited to the particular forms disclosed. Rather, the invention is to cover all modifications, equivalents, and alternatives falling within the scope of the invention as defined by the appended claims.

### DETAILED DESCRIPTION OF THE ILLUSTRATED EMBODIMENTS

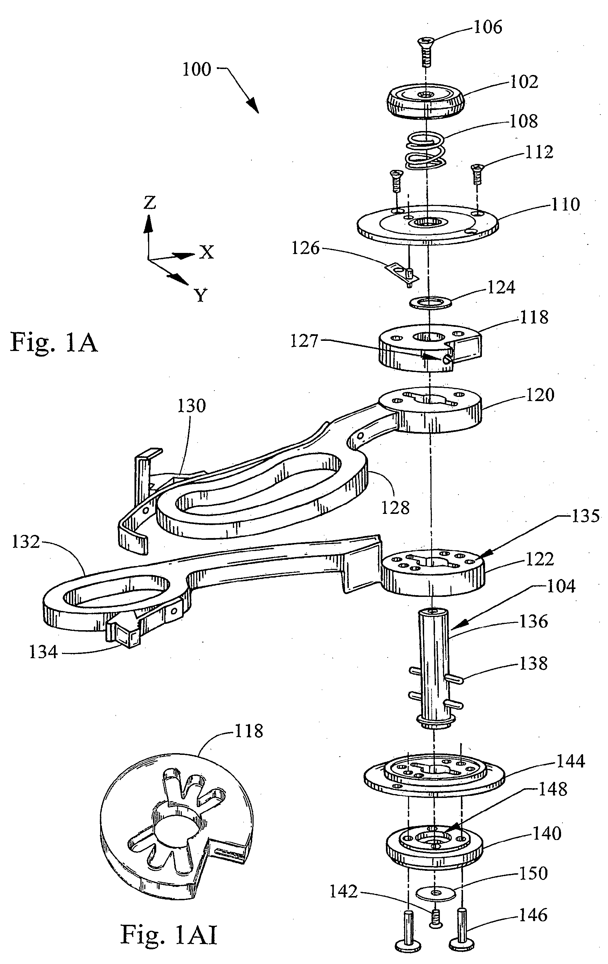

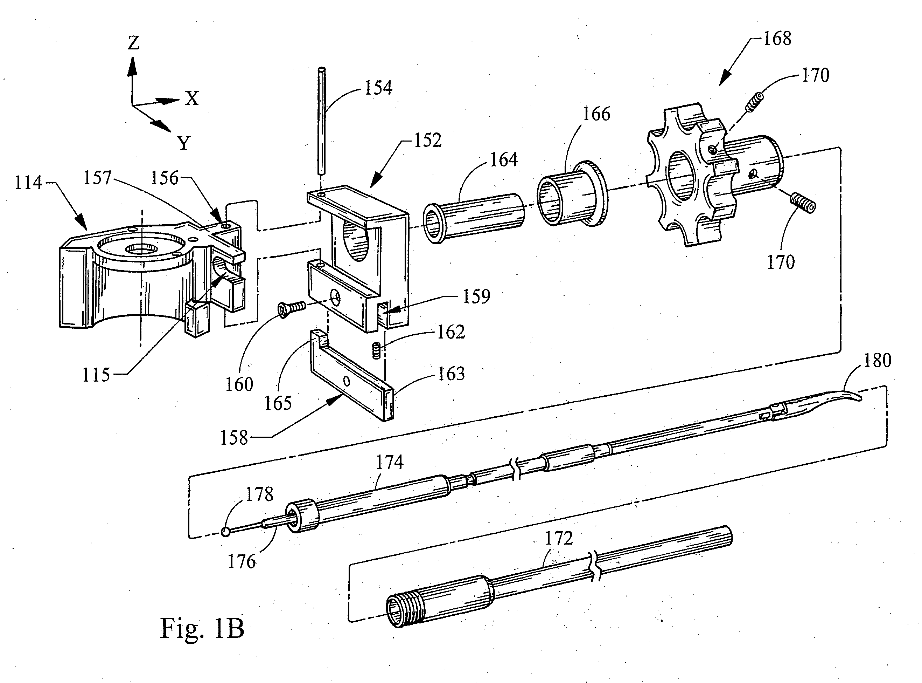

Referring to FIGs. 1A and 1B, a general description of the parts associated with a laparoscopic instrument 100 is provided according to an embodiment of the present invention. A more detailed description of the parts and their associated movements is provided in subsequent drawings. The laparoscopic instrument 100 includes a push button 102 that has a generally cylindrical disk shape. The push button 102 is connected to a winged shaft 104 via a push-button screw 106, which is inserted through a central hole of the push button 102. The push button 102 is adjacent to a spring 108, which includes one end that is in contact with the push button 102 and another end that is in contact with a tool cover 110.

The tool cover 110 is a generally cylindrical plate that includes a central hole and a plurality of tapped periphery holes. The winged shaft 104 protrudes through the central hole of the tool cover 110 toward the push button 102. Two connecting screws 112 connect the tool cover 110 to a housing 114.

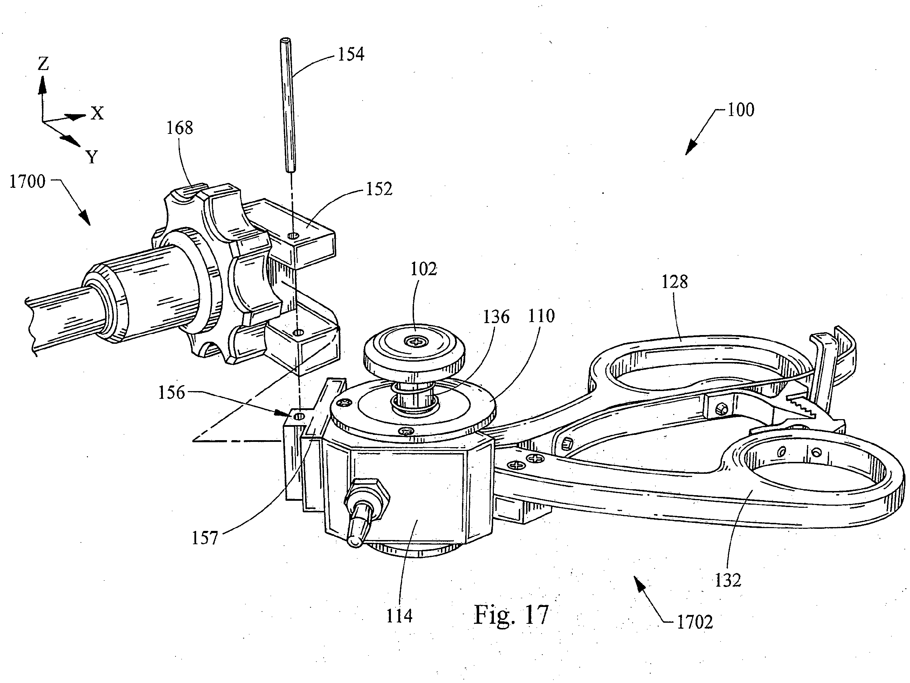

The housing 114 includes a drum receiving portion 116 for accommodating at least in part a tool drum 118, a trigger drum 120, and a fixing drum 122, each of which is located adjacent to one another as shown. The housing 114 further includes a ball-receiving slot 115 for allowing pivoting movement of the housing 114, as described in more detail below in reference to FIGs. 17-19.

Referring to FIG. 1AI, the tool drum 118 is illustrated as being generally cylindrical, having a central through-hole, which is cylindrically shaped, and including a plurality of slots through which the winged shaft 104 protrudes. Although the plurality of slots is shown having three slots, alternatively, any number of slots may be used. The slot height extends only through part of the tool drum 118 (i.e., the slots are not through-slots). For example, the slot height is half the height of the tool drum 118. In alternative embodiments, the slot height extends through the entire tool drum 118. In yet other alternative embodiments, the central hole can have any other three-dimensional shape, e.g., a partial toroid, for receiving the winged shaft 104 therethrough. As explained in more detail below, the slots of the tool drum 118 engage the winged shaft 104 for securing the tool drum 118 to the fixing drum 122 in any one of a plurality of positions.

A drum washer 124 and a plug 126 is located between the tool drum 118 and the housing 114. The tool drum 118 includes a ball-receiving hole 127 along its periphery as shown.

The trigger drum 120 is generally cylindrical and is attached to a trigger handle 128 that includes a latching mechanism 130. The trigger drum 120 is attached to the trigger handle 128 directly or through a mechanical linkage. The trigger drum 120 includes a central slotted hole having substantially the same shape and dimensions as the slotted hole of the tool drum 118 through which the winged shaft 104 protrudes. The fixing drum 122 is generally cylindrical and is attached to a fixing handle 132 that includes a locking part 134 for the latching mechanism 130. The fixing drum 122 is attached to the fixing handle 132 directly or through a mechanical linkage. The fixing drum 122 includes a central slotted hole having substantially the same shape and dimensions as the slotted holes of the tool drum 118 and the trigger drum 120 through which the winged shaft 104 protrudes. The fixing drum 122 further includes a plurality of fixing holes 135 for securing the fixing drum 122 as described in more detail below.

The winged shaft 104 includes a generally cylindrical shaft 136 and a plurality of winglets 138, which are arranged in two symmetrical pairs along the shaft 136. The winglet end of the winged shaft 104 is attached to a locking plate 140 via a locking screw 142. In alternate embodiments, the winglets 138 can be splines or parts thereof, keys, or pins.

A fixing cover 144 is located along the winged shaft 104, between the locking plate 140 and the fixing drum 122. The fixing cover 144 includes a central slotted hole having substantially the same shape and dimensions as the slotted holes of the tool drum 118, the trigger drum 120, and the fixing drum 122 through which the winged shaft 104 protrudes. In addition, the fixing cover 144 includes a plurality of push-pin receiving holes through which corresponding push pins 146 are inserted. The push pins 146 protrude through the locking plate 140, the fixing cover 144, and the fixing holes 135 to secure the fixing drum 122 to the locking plate 140.

The locking plate 140 includes a recessed groove 148 for receiving the winged shaft 104 and a plurality of push-pin receiving holes through which corresponding push pins 146 are inserted. A locking washer 150 is inserted between the head of the locking screw 142 and the locking plate 140.

Turning now to FIG. 1B, the housing 114 is pivotably connected to a hinge 152 via a hinge pin 154, which is inserted through a plurality of hinge pivot holes 156. The housing 114 is attached to the hinge 152 at a housing pivoting portion 157, which is inserted in a hinge slotted area of the hinge 152.

The hinge 152 includes a locking lever 158, which is attached to the hinge 152 via a lever screw 160. The locking lever 158 is inserted in a lever slot 159, which is located at a bottom end of the hinge 152. A lever spring 162 is positioned within the lever slot 159 for maintaining the locking lever 158 in a closed position. The locking lever 158 includes an actuating end 163 and a locking end 165. The actuating end 163 is actuated by urging the locking lever 158 toward the lever spring 162 to unlock the hinge 152 from a closed position to an open position, as described in more detail below in reference to FIGs. 17-19. When the locking lever 158 is pressed, it rotates around the axis of the lever screw 160 such that the locking end 165 causes the hinge 152 to pivot about the axis of the hinge pin 154 (the Z-axis).

A long bearing 164 and a short bearing 166 are used to rotatably attach a knob 168 to the hinge 152. A plurality of set-screws 170 are screwed into the knob 168 for retaining the long bearing 165 and the short bearing 166 relative to the knob 168.

A knob extension or sleeve 172 is attached to the knob 168 using a threaded end of the knob extension 172. The knob extension 172 is a hollow shaft (or sleeve) that is used for accommodating a tool holder 174, which is inserted into the hollow of the knob extension 172. The tool holder 174 is a hollow shaft that accommodates a tool 176, which includes a ball 178 at an insertion end and a scissors device 180 at an operating end. The tool 176 is inserted into the tool holder 174, as shown. According to the shown embodiment, the scissors device 180 is a three-member claw device. The outer surface of the sleeve 172 may be composed of or coated with an insulating material, such as Teflon, to electrically insulate the operator of the instrument 100 from the sleeve 172 when using an electric tool such as a cauterizing tool. For example, the sleeve 172 is wrapped with a Teflon shrink tube.

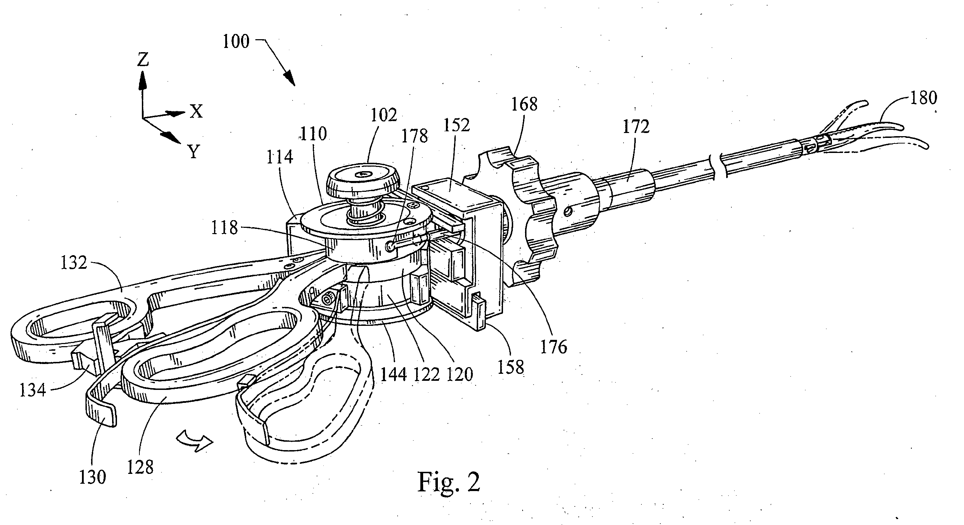

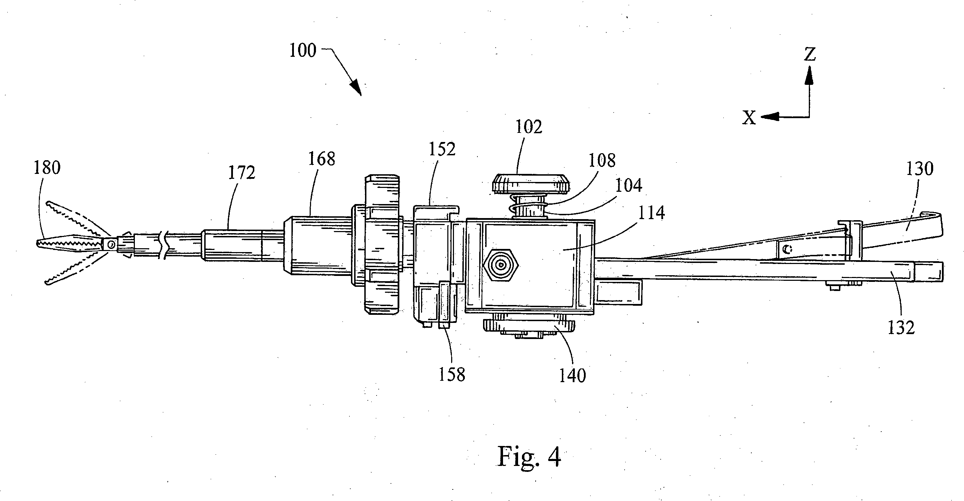

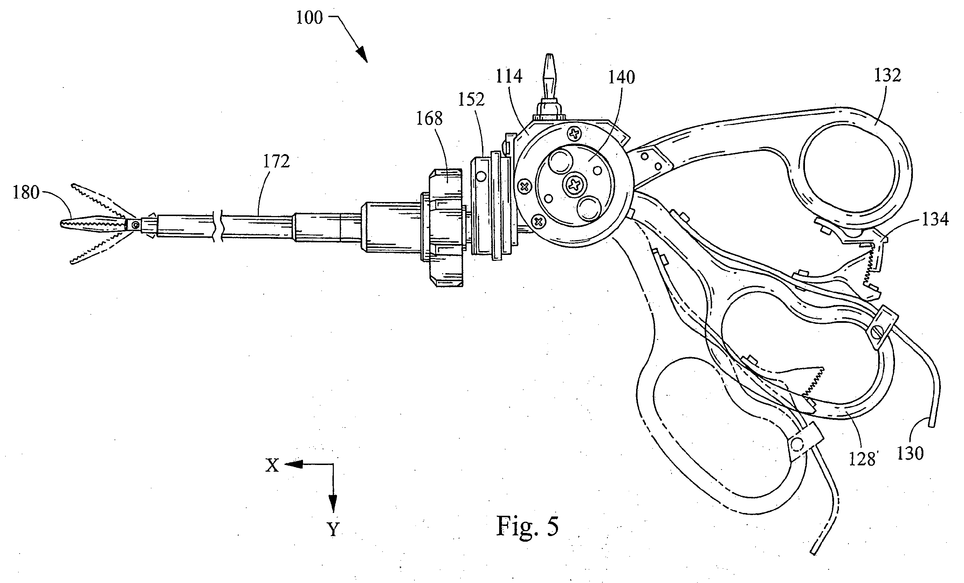

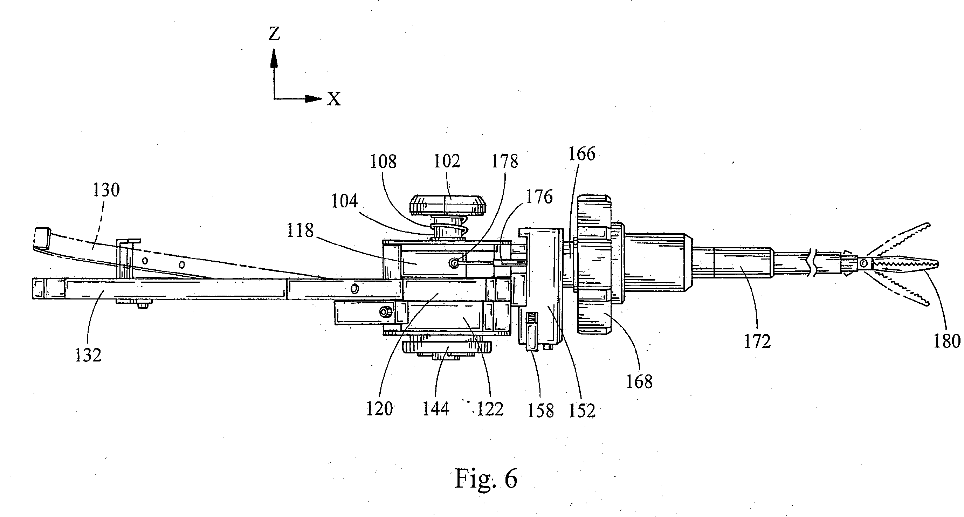

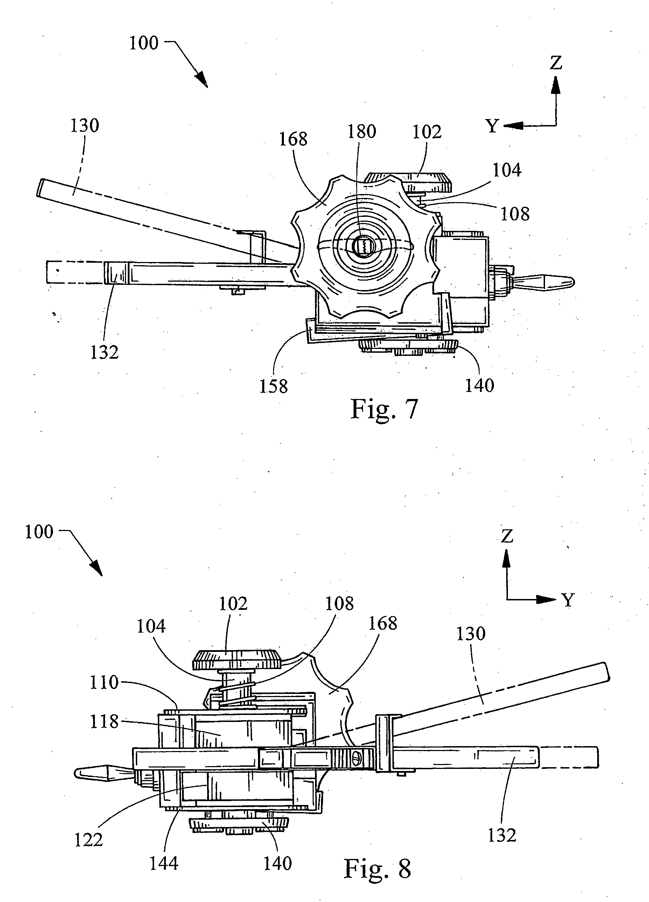

FIGs. 2-8 show various views of the laparoscopic instrument 100 in an assembled form and depict representative movements of the trigger handle 128. The tool drum 118, the trigger drum 120, and the fixing drum 122 are assembled together with the housing 114. The trigger handle 128 and the fixing handle 132 are shown in a locked position, which is described in more detail below. The housing 114 and the hinge 152 are shown in a closed position, and the ball 178 is received by the ball-receiving hole 127 of the tool drum 118.

As represented by the phantom lines, the trigger handle 128 is rotated relative to the fixing handle 132 in a counter clock-wise direction (from the locked position) to open the scissors device 180 at the operating end of the tool 176. In general, the rotation of the trigger handle 128 causes the rotation of the tool drum 118, which in turn causes the linear movement of the tool 176. The linear movement of the tool 176 causes an opening/closing movement for the scissors device 180. The relationship between the three drums 118, 120, 122 (also referred to as the drum sandwich assembly) is described in more detail below.

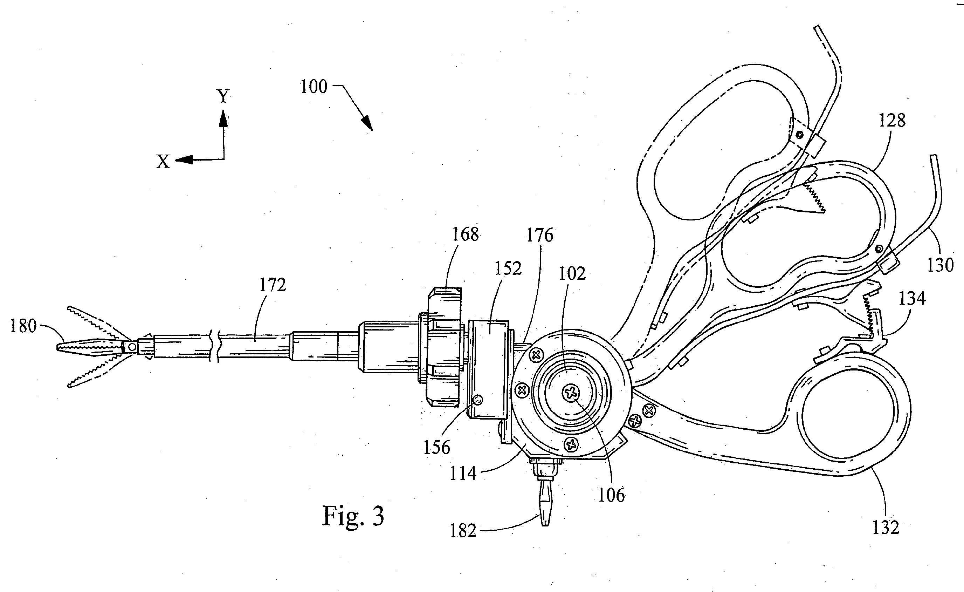

In addition, as best seen in FIG. 3, the position of the shaft 136 (represented by the push-button screw 106) can be aligned with the hinge pivot hole 156 or can be different than the position of the hinge pivot hole 156. For example, the center of the shaft 136 can be at the same distance in the Y-axis direction from the X-axis of the tool 176 as the hinge pivot hole 156. Alternatively, the distance between the center of the shaft 136 and the X-axis of the tool 176 can be smaller or greater than the distance between the hinge pivot hole 156 and the X-axis of the tool 176.

An electrical probe 182 is protruding from and is attached to the housing 114. The electrical probe 182 is electrically coupled to the tool 176 (such as a cauterizing tool) to supply electrical current from an external power supply. For example, electrical current is supplied via the electrical probe 182 to an electrocautery tool 176 for cauterizing organ tissue during a surgical procedure. Alternatively, a hole or plug is formed in the housing 114 for receiving an electrode therein.

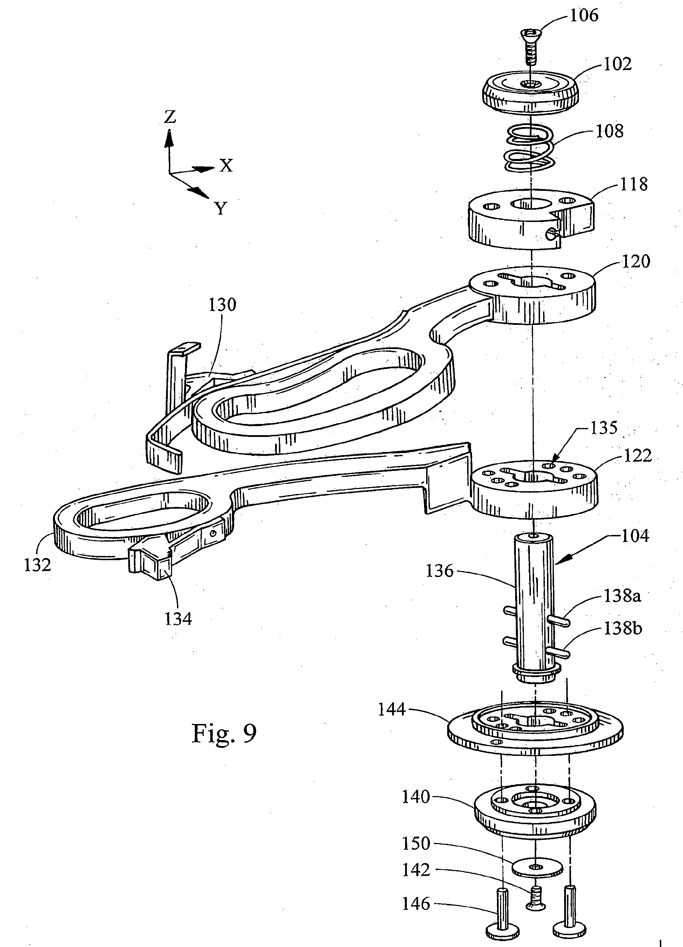

Referring to FIGs. 9-11, the tool drum 118, the trigger drum 120, and the fixing drum 122 are sandwiched between the tool cover 110 (located at the top, adjacent to the tool drum 118) and the fixing cover 144 (located at the bottom, adjacent to the fixing drum 122). The push button 102 is located at the top of the drum sandwich assembly - near the tool cover 110 - and the locking plate 140 is located at the bottom of the drum sandwich assembly - near the fixing cover 144. The shaft 136 protrudes through each of the fixing cover 144, the fixing drum 122, the trigger drum 120, the tool drum 118, and the tool cover 110. The shaft 136 is attached via the locking screw 142 to the locking plate 140 and via the push-button screw 106 to the push button 102. The shaft 136 also protrudes through the spring 108.

The winglets 138 attached to the shaft 136 are adapted to protrude only through corresponding slots of the tool drum 118, the trigger drum 120, the fixing drum 122, and the fixing cover 144. Depending on whether the push button 102 is in a depressed or undepressed position, the winglets 138 protrude through only some of the tool drum 118, the trigger drum 120, the fixing drum 122, and the fixing cover 144. Depending on the position of the winglets 138, the rotatable movement of the trigger drum 120 is locked with respect to either the tool drum 118 or the fixing drum 122.

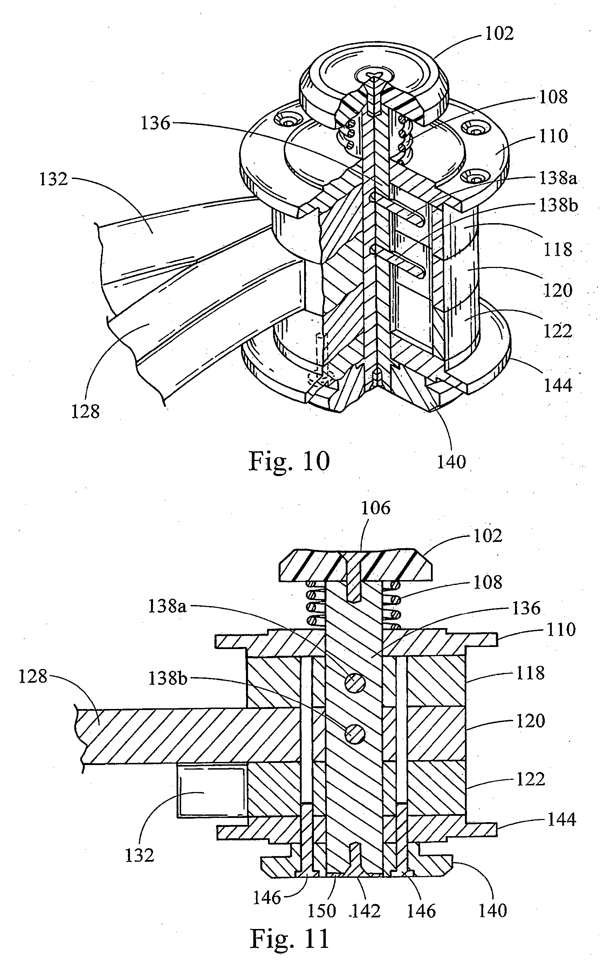

The winglets 138 include a pair of top winglets 138a and a pair of bottom winglets 138b. As shown in FIGs. 10-11, the push button 102 is in an un-depressed position in which the top winglets 138a protrude through the tool drum 118 and the trigger drum 120. In the un-depressed position, the rotatable movement of the trigger drum 120 is fixed with respect to the tool drum 118. If the push button 102 is in a depressed position, the top winglets 138a rotate within the trigger drum 120 (where the bottom winglets 138b are located in the un-depressed position), and the bottom winglets 138b rotate within the fixing drum 122. In the depressed position, the rotatable movement of the trigger drum 120 is fixed with respect to the fixing drum 122. The novel arrangement according to the present invention allows the handles 128, 132 to be rotated regardless of the position of the trigger drum 120 relative to the tool drum 118. This aspect advantageously allows the surgeon to manipulate the handles 128, 132 in any drum position. According to the present invention, instead of having to twist or contort the surgeon's body in order to access a hard-to-reach area of a patient's inner cavity, the surgeon simply rotates the drum to achieve a new position and can continue to manipulate the handles 128, 132, which control the tool 176 inside the patient's body. It is advantageous for the handles 128, 132 to be manipulatable even as they are rotated together around the shaft 136.

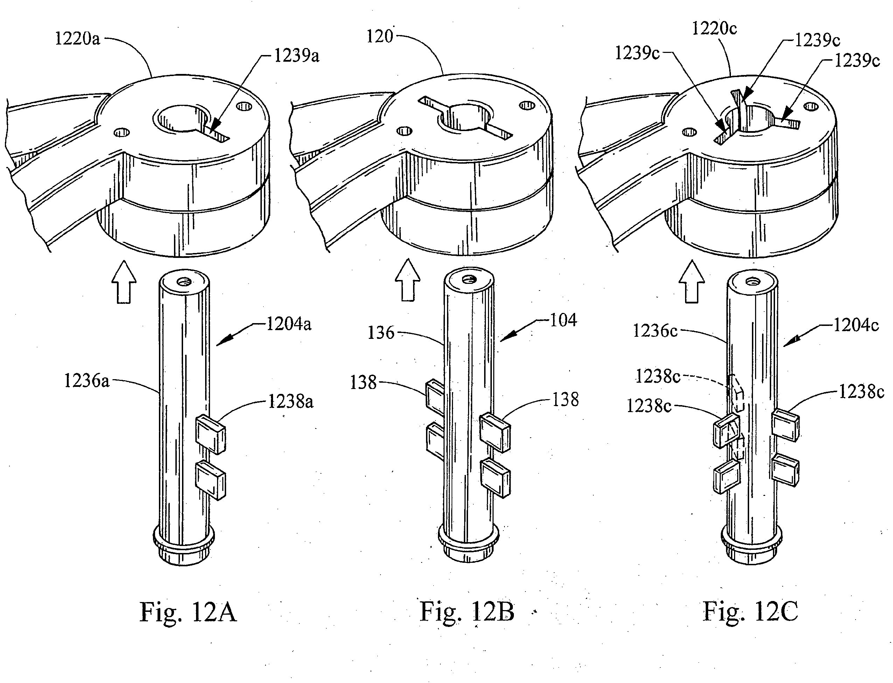

Referring to FIGs. 12A-12C, alternative embodiments of the winged shaft 136 of FIGs. 1-11 are shown depicting three different winglet combinations. For ease of understanding, FIG. 12B shows the winged shaft 104 of FIGs. 1-11, including the shaft 136 and the two sets of winglets 138. In an alternative embodiment, shown in FIG. 12A, a winged shaft 1204a includes a shaft 1236a and a single set of winglets 1238a. To accommodate the single set of winglets 1238a, the holes through which the winged shaft 1204a protrudes (e.g., slotted hole of a trigger drum 1220a) are modified to include a single slot 1239a. Each winglet in the set of winglets 1238a is spaced to lock at most any two drums together when rotated.

In another alternative embodiment, shown in FIG. 12C, a winged shaft 1204c includes a shaft 1236c and three sets of winglets 1238c approximately 120 degrees apart. To accommodate the three sets of winglets 1238c, the holes through which the winged shaft 1204c protrudes (e.g., slotted hole of a trigger drum 1220c) are modified to include three slots 1239c.

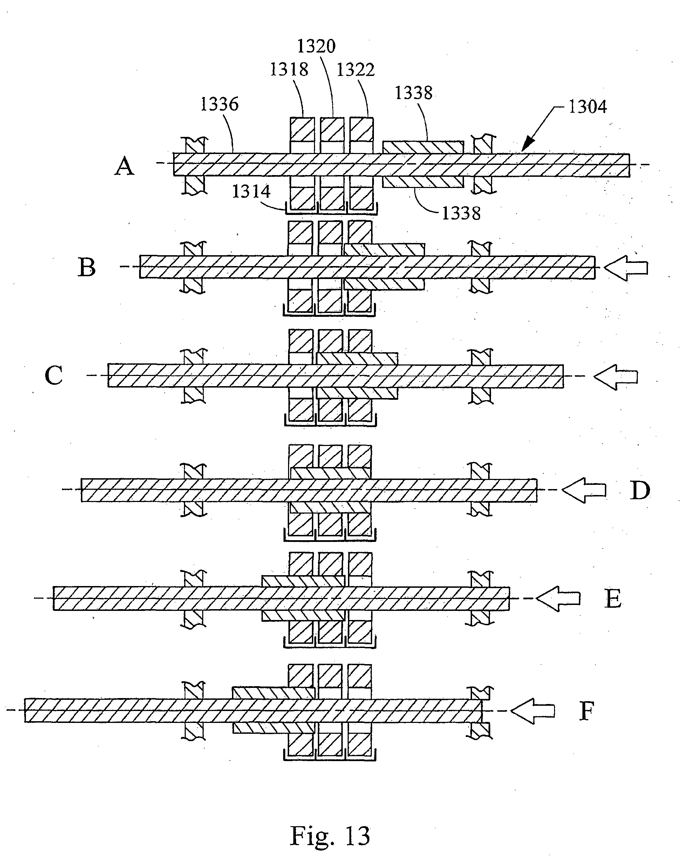

Referring to FIG. 13, a schematic cross-sectional representation illustrates the relationship between a winged shaft 1304 and a plurality of drums, a tool drum 1318, a trigger drum 1320, and a fixing drum 1322, and various positions of the winged shaft 1304 relative to the drums 1318, 1320, 1322. The winged shaft 1304 includes a shaft 1336 and a set of two winglets 1338. From left to right, the drums include the tool drum 1318, the trigger drum 1320, and the fixing drum 1322. The drums 1318, 1320, 1322 are housed within a housing 1314 such that each drum can rotate freely unless fixed in place by the winglets 1338. The drums 1318, 1320, 1322 are fixed from rotational movement when the winglets 1338 protrude through corresponding drum slots. Movement of the winged shaft 1304 interlocks one or more of the drums 1318, 1320, 1322 with respect to each other to achieve a desired rotational combination. For example, as described below, movement of the winged shaft 1304 in any of a plurality of positions A-F achieves any desired rotational combination for the drums 1318, 1320, 1322. As shown in FIG. 13, the winglet and drum combinations can be used to provide a sort of "binary logic" for mechanical devices, such as gears and clutches. The versatility of using the winglets and the drums in accordance with the present invention allows any combination of drum movements to be realized. The concepts of FIG. 13 and related embodiments can be implemented in any mechanical system, including laparoscopic instruments. The present invention expressly contemplates that the lock-and-release embodiments shown and described herein is not limited to laparoscopic instruments.

At position A, the winglets 1338 are positioned to the right of the fixing drum 1322. In this position, each of the drums 1318, 1320, 1322 is free to rotate with respect to each other.

At position B, the winged shaft 1304 is moved toward the drums 1318, 1320, 1322 such that the winglets 1338 are positioned within the fixing drum 1322 only. Accordingly, in this position the fixing drum 1322 is fixed from rotational movement, while the tool drum 1318 and the trigger drum 1320 are free to rotate.

At position C, the winged shaft 1304 is moved further toward the drums 1318, 1320, 1322 such that the wingless 1338 are positioned within both the trigger drum 1320 and the fixing drum 1322. Accordingly, in this position the trigger drum 1320 and the fixing drum 1322 are fixed from rotational movement, while the tool drum 1318 is free to rotate.

At position D, the winged shaft 1304 is moved further toward the drums 1318, 1320, 1322 such that the winglets 1338 are positioned within all three drums. Accordingly, in this position each of the drums 1318, 1320, 1322 is fixed from rotational movement.

At position E, the winged shaft 1304 is moved further toward the drums 1318, 1320, 1322 such that the winglets 1338 are positioned within the tool drum 1318 and the trigger drum 1320. Accordingly, in this position the tool drum 1318 and the trigger drum 1320 are fixed from rotational movement, while the fixing drum 1322 is free to rotate.

At position F, the winged shaft 1304 is moved further toward the drums 1318, 1320, 1322 such that the winglets 1338 are positioned within the tool drum 1318 only. Accordingly, in this position the tool drum 1318 is fixed from rotational movement, while the trigger drum 1320 and the fixing drum 1322 are free to rotate.

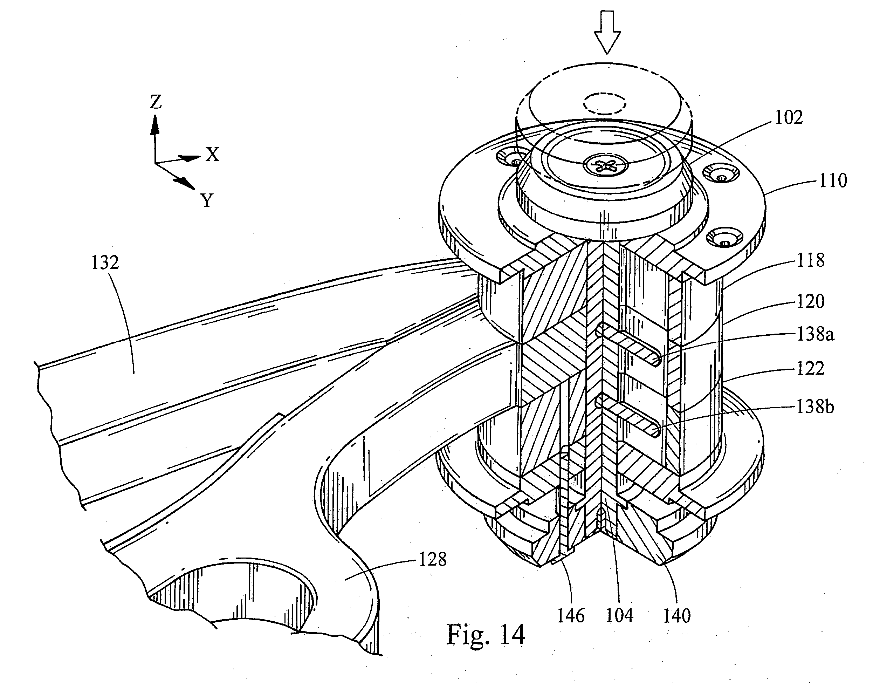

Referring to FIGs. 14-16, a cut-away perspective view of the drums 118, 120, 122 is shown revealing the winged shaft 104 in various positions together with the winglets 138. With reference to these figures, the movement of the winged shaft 104 and of the handles 128, 132 will now be described in more detail. In FIG. 14, the push button 102 is shown in a depressed position, and the handles 128, 132 are shown in a first position. Depressing the push button 102 causes the winged shaft 104 to slide in a direction away from the movement of the push button 102 until the top winglets 138a are located within the trigger drum 120 and the bottom winglets 138b are located within the fixing drum 122. In this configuration, the trigger drum 120 and the fixing drum 122 are fixed or locked together, which in turn locks the handles 128, 132 together. In addition, the locking plate 140 and the push pins 146 are correspondingly urged away from the fixing drum 122, which is now disengaged from the locking plate 140 and the push pins 146.

Accordingly, in the depressed position the trigger drum 120 and the fixing drum 122 are locked with respect to each other. Further, because the fixing drum 122 is now disengaged from the locking plate 140 and the push pins 146, the combination of the trigger drum 120 and the fixing drum 122 is free to rotate around the Z-axis (the axis of the winged shaft 104).

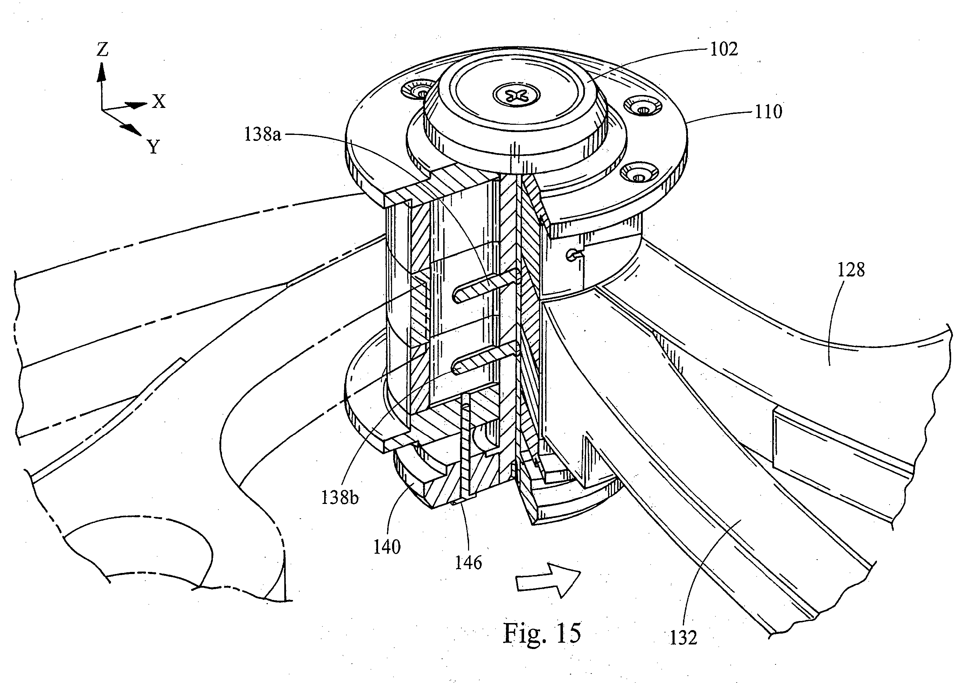

In FIG. 15, the push button 102 remains in the depressed position. However, the handles 128, 132 have been rotated counter clock-wise from the first position to a second position. Thus, the only two components that change their position from the first position to the second position are the trigger handle 128 and the fixing handle 132. For example, the position of the tool drum 118 remains unchanged. By rotating the handles 128, 132 to a new position, while maintaining the position of the tool drum 118, a surgeon using the laparoscopic instrument 100 may be able to achieve a better grasping position for the handles 128, 132 without changing the position of the tool 176 inside a patient and without contorting or twisting the surgeon's body to maintain a comfortable and firm grasp.

As can be seen in FIGs. 1A and 9, the fixing handle 132 is secured to the locking plate 140 by inserting the push pins 146 through the fixing holes 135. Three pairs of fixing holes 135 are shown, and each fixing hole pair represents a different handle position (up to three different positions in the embodiment shown in FIG. 1A). When the push button 102 is depressed, the push pins 146 disengage the fixing holes 135, allowing the fixing drum 122 to freely rotate. The force exerted by the spring 108 allows the surgeon to rotate the fixing drum 122 (and thereby the fixing handle 132) until the push pins 146 "click" into alignment with a different set of fixing holes 135. Although three pairs of fixing holes 135 are shown allowing the fixing handle 132 to be rotated among one of three different positions, fewer or additional fixing holes are contemplated in other embodiments to allow the fixing handle 132 to be rotated among a corresponding number of positions. For example, if four positions are desired, four pairs of fixing holes 135 are formed in the fixing drum 122 and spaced according to each desired position. Although two push pins 146 are shown in FIG. 1A, in other embodiments, a different number of push pins is used instead.

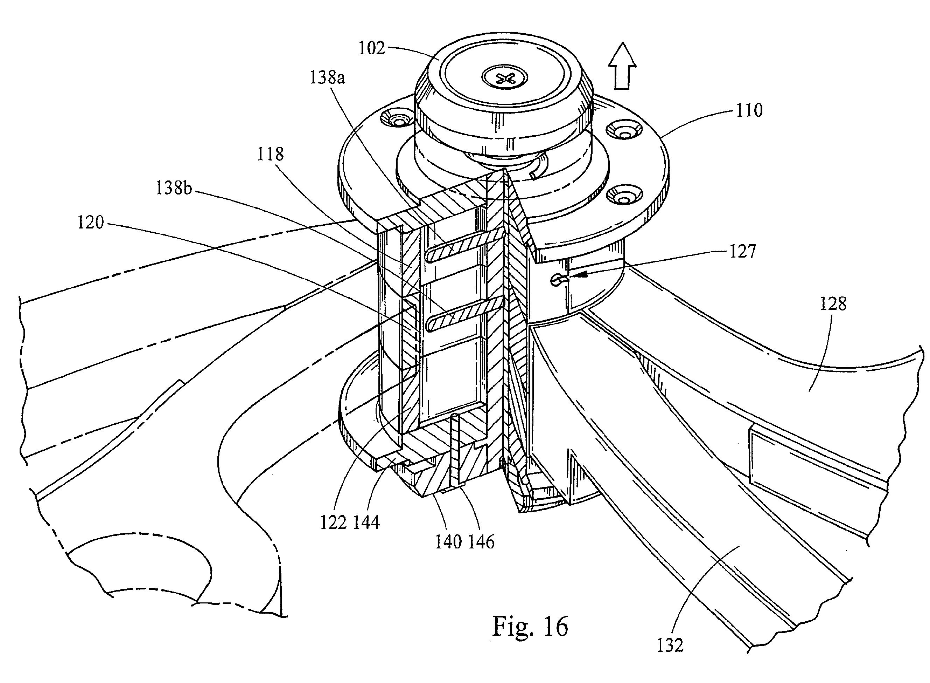

In FIG. 16, the push button 102 is shown in the un-depressed position to engage the fixing drum 122 to the locking plate 140 and the trigger drum 120 to the tool drum 118. The winglets 138 are now located within the tool drum 118 and the trigger drum 120 to secure the tool drum 118 and the trigger drum 120 to each other. The push pins 146 engage the fixing drum 122, fixing the handles 128, 132 in a second position. When the tool drum 118 and the trigger drum 120 are fixed relative to each other, i.e., in the un-depressed position, the trigger handle 128 may be partially rotated. The rotation of the trigger handle 128 causes the rotation of the tool drum 118, which in turn causes the linear movement of the tool 176. The linear movement of the tool 176 allows the surgeon to use the operating end of the tool 176. For example, a counter clock-wise movement of the trigger handle 128 causes the opening of the scissors device 180, while a clock-wise movement of the trigger handle 128 causes the closing of the scissors device 180.

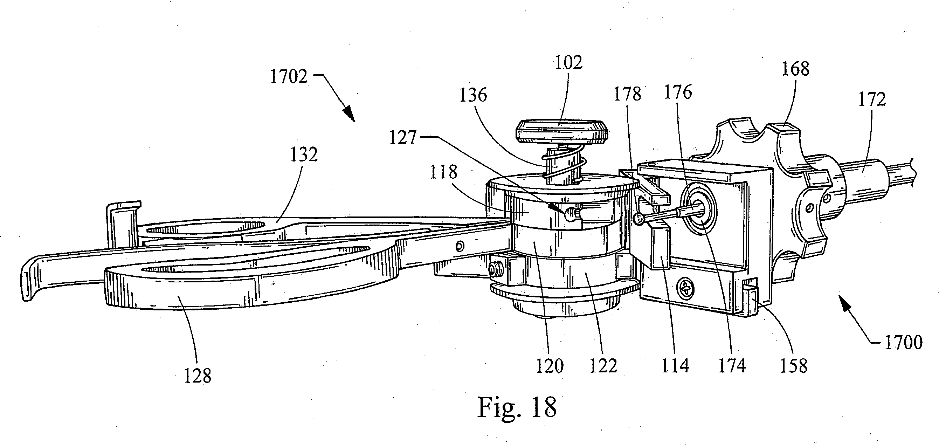

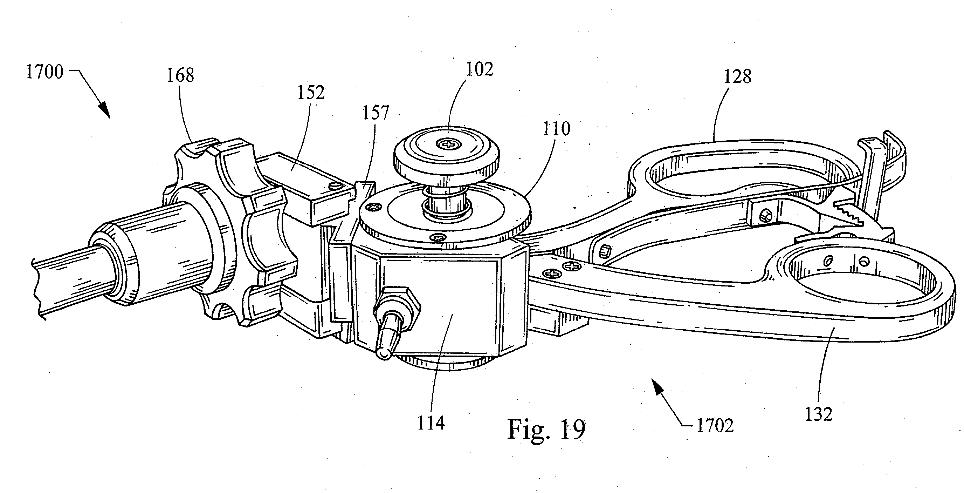

Referring to FIGs. 17-19, there is shown a shotgun subassembly of the laparoscopic instrument 100 in an open "breech" position. The term "shotgun" subassembly refers to the resemblance of the laparoscopic instrument 100 to the breech of a shotgun, which allows the surgeon to replace the laparoscopic tool without removing the instrument 100 from the patient's body. While the instrument 100 is inserted into the patient's body, the shotgun subassembly can be opened and closed like a shotgun to expose one end of the tool for removal and reinsertion. The laparoscopic instrument 100 includes a hinge portion 1700 and a housing portion 1702, which together form the shotgun subassembly having a "breech" that is pivotable about a hinge 152. The hinge portion 1700 generally includes the hinge 152, the tool 176, and the scissors device 180. The housing portion 1702 generally includes the housing 114, the handles 128, 132, and the drums 118, 120, 122. The pivoting of the hinge 152 with respect to the housing 114 of the laparoscopic instrument 100 is described in more detail in connection with FIGs. 18 and 19. In FIG. 17, the hinge 152 is assembled to the housing pivoting portion 157 using the hinge pin 154. The housing 114 pivots about the hinge pin 154 in the Z-axis to provide the opening and/or closing movement of the housing portion 1702 with respect to the hinge portion 1700.

In FIGs. 18 and 19, the hinge portion 1700 is shown in an open position, having been pivoted in a counter clock-wise direction about the Z-axis from the closed position. As the hinge portion 1700 is urged toward the open position, the ball 178 - along with the tool 176 - is retracted from the ball-receiving hole 127 of the tool drum 118. To open the hinge portion 1700, the locking lever 158 is pressed in a direction toward the tool 176 (as described earlier in reference to FIG. 1B) such that the locking end 165 (shown in FIG. 1B) releases the housing pivoting portion 157. As the hinge portion 1700 is urged toward the open position, the ball 178 passes through the ball-receiving slot 115 formed in the housing 114 until the ball 178 exits the ball-receiving slot 115. After moving the hinge portion 1700 into the open position, the surgeon can remove the tool 176 from within the hinge portion 1700 and replace it with another laparoscopic tool without removing any other part of the instrument 100 from the patient's body. Thus, during the tool replacement, the knob extension or sleeve 172 remains inside the patient in a fixed position. In other words, in contrast to prior art laparoscopic instruments, the surgeon is not required to remove the instrument 100 from within the patient in order to replace the tool 176 with another tool. Maintaining the instrument 100 inside the patient while exchanging tools advantageously eliminates the need for the surgeon to search for and find a previously located body part or position.

The location of the ball-receiving hole 127 is found by drawing a circle about the hinge pin 154, whose radius extends to the end of the ball 178 (when the tool 176 is fully inserted into the knob extension 172). Where the circle intersects the tool drum 118 is where the manufacturer should form the ball-receiving hole 127.

In an alternate embodiment, instead of adapting the hinge portion 1700 to swing open, the hinge portion 1700 is adapted to slide open. For example, instead of having the housing 114 rotatable with respect to the hinge 152, the housing 114 slides open with respect to the hinge 152 in, for example, a direction of the Z-axis, to allow the removal and/or insertion of the tool 176.

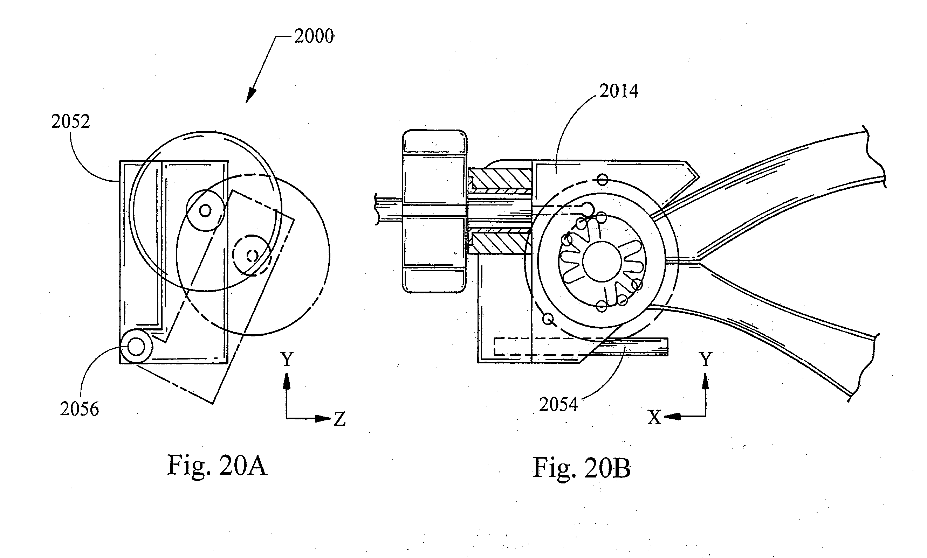

Referring to FIGs. 20A and 20B, an alternative embodiment of the present invention shows a laparoscopic instrument 2000 that includes a housing 2014 and a hinge 2052. The hinge 2052 pivots around an X-axis of the laparoscopic instrument 2000. Specifically, the hinge 2052 pivots around a hinge pin 2052, which is inserted through a hinge pivot hole 2056, with respect to the housing 2014.

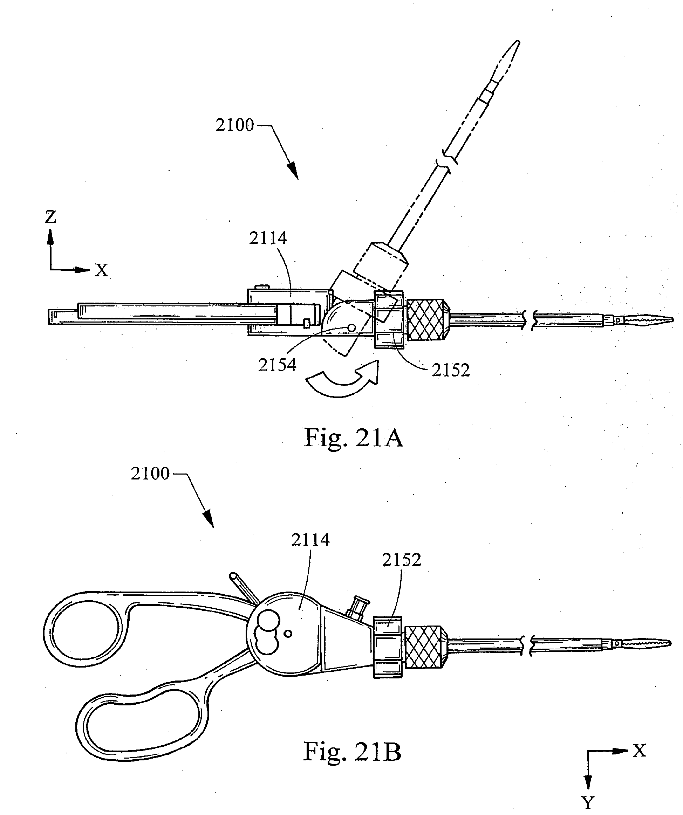

Referring to FIGs. 21A and 21B, an alternative embodiment of the present invention shows a laparoscopic instrument 2100 that includes a housing 2114 and a hinge 2152. The hinge 2152 pivots around a Y-axis of the laparoscopic instrument 2100. Specifically, the hinge 2152 pivots around a hinge pin 2152 with respect to the housing 2114. Slots in the housing 2014 and 2114, respectively, and respective drums will enable the exposed part of each respective shaft and ball to travel into each respective drum.

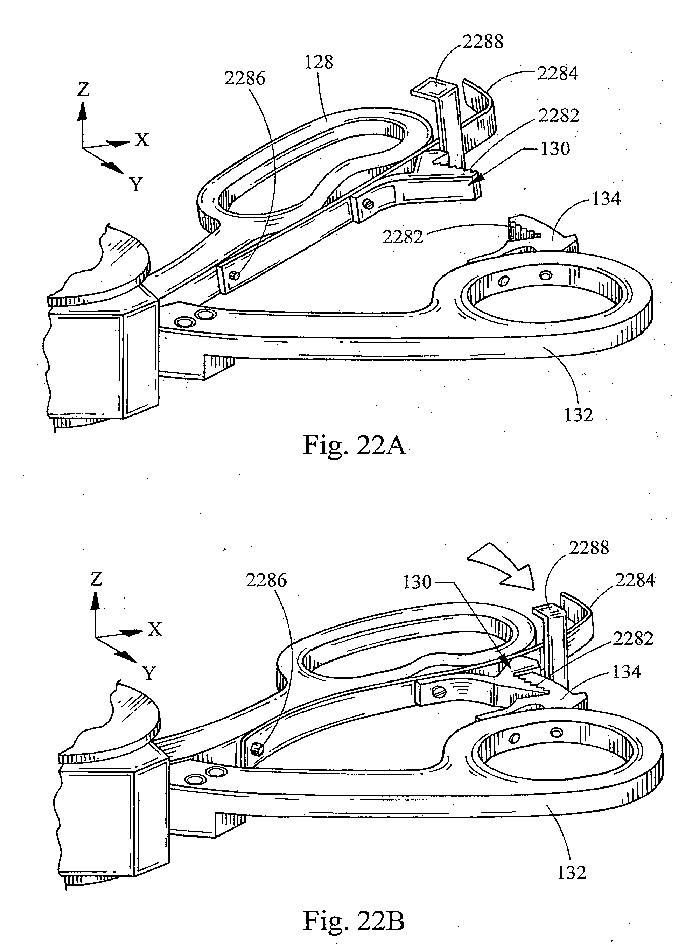

Referring to FIGs. 22A-23B, the locking of the trigger handle 128 with respect to the fixing handle 132 will be described in more detail. In FIG. 22A, the handles 128, 132 are in an open and aligned position relative to one another. In the open position there is no contact between the latching mechanism 130 of the trigger handle 128 and the locking part 134 of the fixing handle 132. The latching mechanism 130 and the locking part 134 include a plurality of corresponding teeth 2282 that are biased so as to lock the handles 128, 132 to each other, as described in more detail below in reference to FIG. 22B.

The trigger handle 128 further includes a latching lever 2284, which is pivotally connected to the trigger handle 128 at a pivoting point 2286, and a lever limiter 2288. The lever limiter 2288 limits the rotational movement of the latching lever 2284 to a distance that is sufficient for disengaging engaged ones of the teeth 2282. A reason for limiting the rotational movement of the latching lever 2284 is to prevent the latching lever 2284 from interfering with the operation of the laparoscopic instrument 100. The latching mechanism 130 is mounted on the latching lever 2284 such that the latching mechanism 130 moves whenever the latching lever 2284 is moved. The aligned position shows the latching lever 2284 parallel to the fixing handle 132 in the X-Y plane.

In FIG. 22B, the handles 128, 132 are shown in a locked position, and the handles 128, 132 are correspondingly in a closed and aligned position. The latching mechanism 130 and the locking part 134 are interlocked via the plurality of corresponding teeth 2282, which are included in each of the latching mechanism 130 and the locking part 134. To lock the handles 128, 132, at least one of the handles 128, 132 is rotated around the Z-axis toward the other one of the handles 128, 132. For example, the trigger handle 128 is rotated in a clockwise direction toward the fixing handle 132. Corresponding ones of the teeth 2282 are engaged via frictional forces to prevent movement of the handles 128, 132 toward an open position. The teeth 2282 are biased to encourage movement of the handles 128, 132 toward one another but to resist movement of the handles 128, 132 away from one another. The ability to lock the handles 128, 132 during surgery advantageously frees the surgeon's hand to carry out other tasks, while leaving the instrument 100 inside the patient's body. It further permits the surgeon to relax the hand gripping the instrument 100 to minimize hand fatigue that can be caused by prolonged grasping and manipulation of the handles 128, 132. Still further, without locking handles, if the surgeon's hand that is grasping the handles 128, 132 were to momentarily relax or lose its grip, the tool 176 may slip or dislodge from a desired position inside the patient's body cavity. When the handles 128, 132 are in the locked position, the tool 176 can be reliably maintained inside the patient. With the handles locked, the surgeon may also rotate them together in accordance with the present invention to a better position without disturbing the position of the tool 176 inside the body cavity.

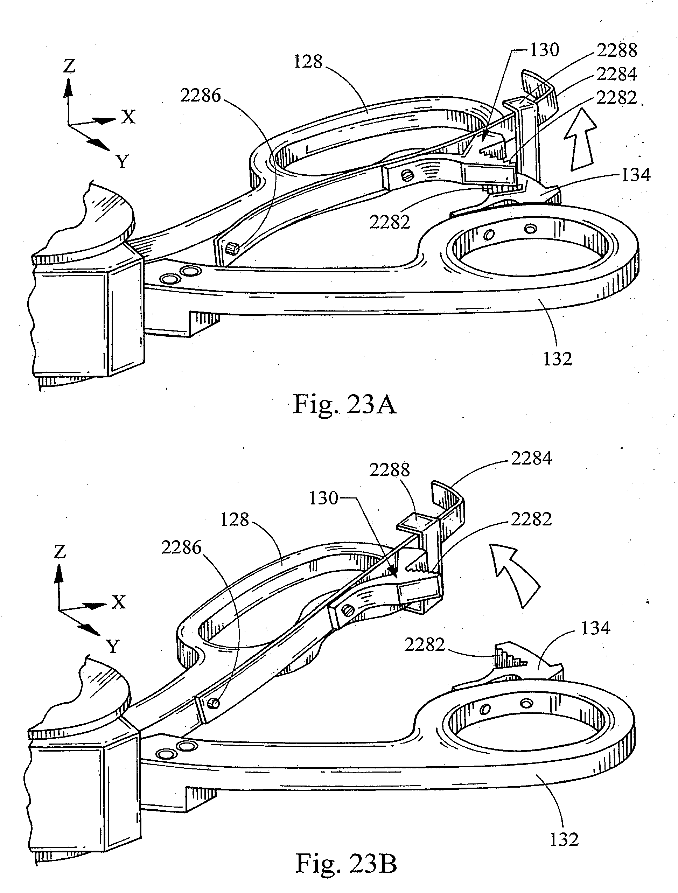

In FIG. 23A, the handles 128, 132 are shown in a closed and offset position. The handles 128, 132 are fixed with respect to the Z-axis as the latching lever 2284 is urged in the Z-axis direction to unlock the latching mechanism 130 from the locking part 134. When the latching mechanism 130 is moved in the Z-axis direction away from the locking part 134, via movement of the latching lever 2284, engaged ones of the teeth 2282 disengage, causing the trigger handle 128 to unlock from the fixing handle 132.

In FIG. 23B, the handles 128, 132 are shown in an open and offset position. After the trigger handle 128 is moved in the Z-axis direction (as shown in FIG. 23A) away from the fixing handle 132, the trigger handle 128 is rotated in a counter-clockwise direction around the Z-axis. To position the latching lever 2284 in the initial open and aligned position of FIG. 22A, the latching lever 2284 must be urged in the Z-axis direction toward the trigger handle 128 in order to position the latching lever 2284 in the same X-Y plane as the fixing handle 132. Now, the trigger handle 128 is ready to be locked relative to the fixing handle 132.

Preferably, the latching lever 2284 is positioned to be manipulatable by the surgeon with a single finger, such as with the pinky finger of the hand grasping the handles 128, 132. In this respect, the surgeon is not required to remove the hand from the handles 128, 132 in order to lock or unlock them. In operation, the surgeon simply moves the latching lever 2284 with the pinky finger, which is typically not positioned within the handle 128 as are the ring and middle fingers.

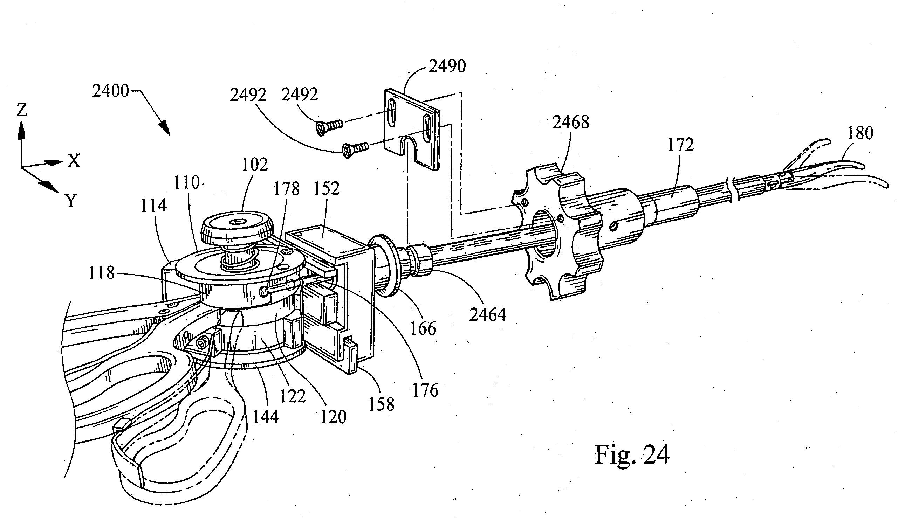

Turning now to FIG. 24, a partially exploded surgical instrument 2400 is shown arranged to permit removal of the surgical instrument 2400 from a sleeve 172 that remains in situ as the surgical instrument 2400 is removed from the patient's body. The surgical instrument 2400 can be a laparoscopic instrument in some embodiments or an arthroscopic instrument in other embodiments. In the embodiment shown in FIG. 24, the surgical instrument includes a knob 2468 attached to the knob extension (or sleeve) 172 through which the tool 176 is inserted and a locking plate 2490 that is shaped to fit within a groove formed in a long bearing 2464 as shown. The embodiment shown in FIG. 24 differs from the embodiment shown in FIGS. 18-19, for example, in that in FIG. 24, the surgical instrument 2400 is removable from the sleeve 172 without rotating the surgical instrument 2400 into a breech position as described in connection with FIGS. 18 and 19. In both embodiments, the sleeve remains in the body as the tool 176 is removed; in FIGS. 18 and 19, the tool 176 is removed by rotating the hinge portion 1700 as described into an open position and then grasping the tool 176 by the exposed ball 178, whereas in FIG. 24, the entire surgical instrument 2400 (except for the locking plate 2490, the sleeve 172, and the knob 2468) is removed by urging the locking plate 2490 away from the groove formed in the long bearing 2464.

The locking plate 2490 is secured to the knob 2468 by a pair of screws 2492, which pass through elongated slots in the locking plate 2490, permitting movement of the locking plate 2490. When the locking plate 2490 is in a locked position, the sleeve 172 is coupled to the housing 114 through the cooperation of the short bearing 166 (which is shown in FIG. 24 partially exploded for ease of illustration), the long bearing 2464, and the locking plate 2490. When the locking plate 2490 is urged from the locked position to an open or unlocked position, the locking plate 2490 exits the groove formed in the long bearing 2464 to permit separation or decoupling of the knob-and-sleeve assembly 2468, 172 from the remainder of the surgical instrument 2400. The sleeve 172 remains in the patient's body, allowing the surgeon to quickly locate the internal part of the body on which the surgeon is operating when a new tool is inserted through the sleeve 172.

After a new tool is inserted into the sleeve 172 and the locking plate 2490 is locked over the groove in the long bearing 2464, the sleeve 172 is once again secured to the remainder of the surgical instrument 2400 and forms an integral part thereof. In other words, the separation of the sleeve 172 from the remainder of the surgical instrument 2400 depends on the position of the locking plate 2490.

Note that in FIG. 24, the surgical instrument 2400 is also adapted to exchange tools by way of the "shotgun" technique described in connection with FIGS. 18 and 19, giving the surgeon two options for exchanging tools. For example, after the tool is removed by unlocking the locking plate 2490, the hinge portion 1700 can be rotated (after depressing the locking lever 158 to release the hinge portion 1700) into an open "breech" position to expose the ball 178 of the tool 176, which can be grasped by the operator to remove the tool 176 and exchange it for another. In other embodiments, the surgical instrument 2400 does not have a hinge portion 1700, and permits tool exchange by releasing the locking plate 2490. Once removed from the patient's body, the tool 176 can be further decoupled from the surgical instrument 2400.

The locking plate 2490 can be configured in any number of ways in order to facilitate a quick-release lock. For example, in some embodiments, the locking plate 2490 can have a spring-loaded mechanism that unlocks the locking plate 2490 by pushing down on the plate 2490 causing the plate 2490 to spring away from the groove formed in the long bearing 2464. A specific embodiment is shown in FIG. 24, however, the present invention contemplates other mechanisms for releasably locking the sleeve 172 from the remainder of the instrument. In all embodiments, the sleeve 172 can remain within the patient's body as the surgical instrument is withdrawn therefrom through the cannula/trocar unit anchored at the incision interface. The tool 176 can be withdrawn through the sleeve 172 without removing any other part of the surgical instrument or the tool 176 can be withdrawn together with the instrument as the sleeve 172 remains in situ. As mentioned above, the sleeve 172 helps the surgeon to locate quickly the location of the operation site when a new tool is inserted through the sleeve 172. By keeping the sleeve that operates as a "placeholder" within the patient's body, the surgeon no longer needs to regain the "feel" lost by removal of the tool and relocate the operation site.

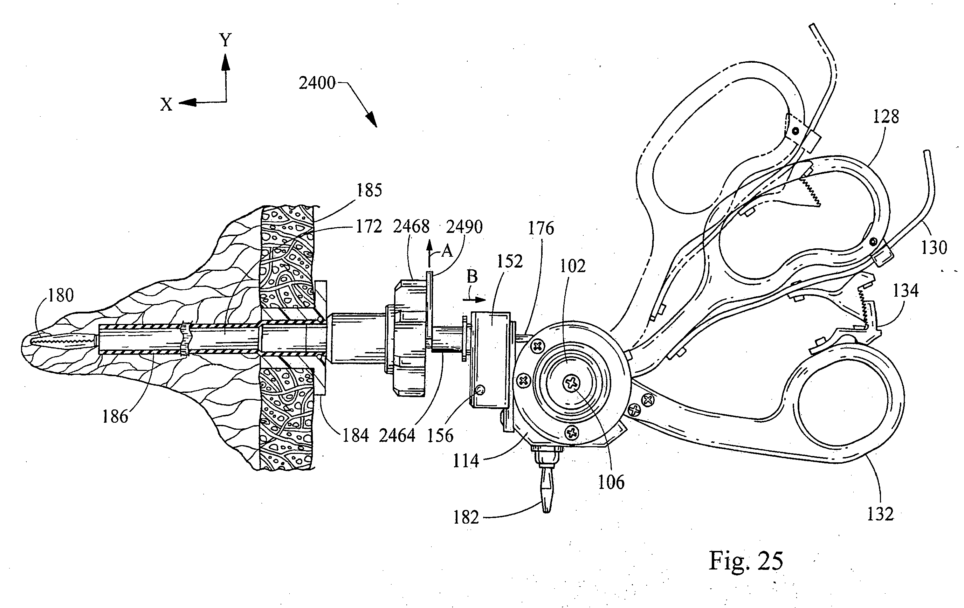

Turning now to FIG. 25, the surgical instrument 2400 is shown partially inserted into a patient's body 185 by way of a cannula/trocar unit 184. The locking plate 2490 has been moved to the unlocked position in the direction of arrow A, releasing the surgical instrument 2400 from the sleeve 172, and the surgical instrument 2400 has been partially retracted from the sleeve 172 in the direction of arrow B. An insulating layer or coating 186 is disposed or formed within the hollow shaft of the sleeve 172 to prevent electrocution of the operator of the instrument 100 when applying an electric current to the tool 180 for use in, for example, a cauterizing procedure. As mentioned above, the electrical probe 182 is used for electrically connecting the tool 180 to a power source. The insulating layer 186 or coating can include Teflon, for example.

At least some of the parts described above in reference to FIGs. 1A-25 are injection-molded parts, which are precision molded with hot-oil or water molds using high-strength, graphite-, glass-, or carbon-filled plastics such as PEEK™ (polyetheretherketone), Ultem® (polyetherimide), Grivory®, or RADEL® R (polyphenylsulfone). The injection-molded parts include single cavity molds and family molds. For example, some of the molded parts can be cold runner molds.

Although the foregoing embodiments have been described in connection with a laparoscopic instrument 100, the present invention is equally applicable to an arthroscopic instrument.

While the invention is susceptible to various modifications and alternative forms, specific embodiments thereof have been shown by way of example in the drawings and herein described in detail. It should be understood, however, that it is not intended to limit the invention to the particular forms disclosed, but on the contrary, the intention is to cover all modifications, equivalents, and alternatives falling within the scope of the invention as defined by the appended claims.

## Claims

1. A laparoscopic instrument, comprising
a shaft (104, 1204a, 1204c, 1304) including a plurality of winglets (138, 1238c, 1338) disposed along a length of the shaft,
a first handle (128, 132) pivotally rotatable among at least two lockable positions, and
a first drum (120, 122, 1220a, 1220c, 1320, 1322) attached to the first handle (128, 132), the shaft passing through the first drum, and the first drum being rotatable around the shaft to cause the first handle to be pivotally rotatable among the at least two lockable positions,
**characterized by**
a second drum (120, 122, 1220a, 1220c, 1320, 1322) attached to a second handle (128,132) pivotally rotatable among the least two lockable positions with the first handle (128, 132), and further
**characterized in that**
the shaft (104, 1204a, 1204c, 1304) passes through the first drum and second drum (120, 122);
the first drum has at least one slot extending through at least part of the first drum;
the second drum has at least one slot extending through at least part of the second drum (120, 122, 1220a, 1220c, 1320, 1322); and
the plurality of shaft winglets (138, 1238c, 1338) and said drum slots are positioned such that said shaft is movable through said first and second drums between one position in which said winglets protrude through respective slots of the first and second drums to lock together the first drum (120, 122, 1220a, 1220c, 1320, 1322) and the second drum (120, 122, 1220a, 1220c, 1320, 1322) while the shaft is rotated among the at least twolockable positions, and another position in which none of the plurality of winglets protrude into said at least one slot of said first drum and in which at least one of said winglets protrudes through at least one slot of the second drum to permit said first drum to rotate independently of said second drum.

2. The laparoscopic instrument of claim 1, wherein the shaft (104, 1204a, 1204c, 1304) passing through the second drum (120, 122), the second drum being rotatable around the shaft to cause the second handle (128,132) to be pivotally rotatable among the at least two lockable positions.

3. The laparoscopic instrument of claim 1, further comprising: a tool drum (118,1318) coupled to the first drum and the second drum; the tool drum having at least one slot extending through at least part of the tool drum, the shaft (104, 1204a, 1204c, 1304) passing through the first drum, the second drum, and the tool drum so as to be rotatable among the at least two lockable positions.

4. The laparoscopic instrument of claim 3, wherein the plurality of winglets (138, 1238c, 1338) and the drum slots are positioned such that said shaft is movable through said first drum, said second drum and said tool drum, to a position in which said winglets protrude through respective slots of at least two of the first drum, the second drum and the tool drum to lock together said at least two of the first drum (120, 122, 1220a, 1220c, 1320, 1322), the second drum (120, 122, 1220a, 1220c, 1320, 1322) and the tool drum (118,1318) while the shaft is rotated among the at least two lockable positions.

5. The laparoscopic instrument of claim 1 further comprising: a housing (114, 1314); said, shaft (104, 1204a, 1204c, 1304) passing through the first drum (120, 122, 1220a, 1220c, 1320, 1322) for securing the first drum along a central axis of the housing, the shaft (104, 1204a, 1204c, 1304) being inserted through the first drum; and a push-button connected to the shaft for moving the shaft between a first position and a second position, the first position corresponding to one of the at least two lockable positions, the second position corresponding to an unlocked position.

6. The laparoscopic instrument of claim 5, wherein the first handle (128,132) is movable between the at least two lockable positions when the shaft is in the second position.

7. The laparoscopic instrument of claim 1 further comprising: a tool (176) having a ball end and a tool end, the ball end being removably connected to a tool drum (118,1318) in any of the at least two lockable positions.

8. The laparoscopic instrument of claim 7, further comprising the drum (120. 122, 1220a, 1220c, 1320, 1322) attached to the handle (128,132), the tool drum (118, 1318) being secured to the drum in at least one of the at least two lockable positions.

9. The laparoscopic instrument of claim 1 having a transverse axis along which a tip is disposed, wherein said first handle (128,132) is a fixing handle (132), wherein said first drum is a fixing drum (122, 1322) and further comprising a trigger handle (128) pivotally rotatable among at least two lockable positions about an axis orthogonal to the transverse axis.

10. The laparoscopic instrument of claim 9, wherein said second drum comprises a trigger drum (120, 1220a, 1220c, 1320) attached to the trigger handle (128); and further comprising a tool drum (118, 1318) rotationally secured to the trigqer drum (128) in the at least two lockable positions; said fixing drum (122, 1322) being attached to said fixing handle (132, the fixing drum being rotationally secured to the trigger drum when the trigger drum is in an unlocked position.

11. The laparoscopic instrument of claim 10, wherein said winged shaft (104, 1204a, 1204c, 1304) is for axially securing the trigger drum (120, 1220a, 1220c, 1320), the tool drum (118, 1318), and the fixing drum (122, 1322) to each other, the tool drum having at least one slot extending through at least part of the tool drum, wherein the pluralité of shaft winglets (138, 1238c, 1338) and the slots are positioned such that the winged shaft is movable between a first position and a second position, the shaft winglets protruding through respective slots of the tool drum (118,1318) and the trigger drum (120, 1220a, 1220c, 1320) to rotationally secure the tool drum with respect to the trigger drum in the first position, and the shaft winglets protruding through respective slots of the trigger drum (120, 1220a, 1220c, 1320) and the fixing drum (122, 1322) to rotationally secure[d] the trigger drum with respect to the fixing drum in the second position, the first position corresponding to one of the at least two lockable positions.

## Patentansprüche

1. Laparoskopisches Instrument, das Folgendes umfasst:
eine Welle (104, 1204a, 1204c, 1304) mit mehreren Flügelchen (138, 1238c, 1338), die über eine Länge der Welle angeordnet sind,
einen ersten Griff (128, 132), der zwischen wenigstens zwei verriegelbaren Positionen schwenkbar ist, und
eine erste Walze (120, 122, 1220a, 1220c, 1320, 1322), die am ersten Griff (128, 132) angebracht ist, wobei die Welle durch die erste Walze läuft und die erste Walze sich um die Welle drehen kann, um zu bewirken, dass der erste Griff zwischen den wenigstens zwei verriegelbaren Positionen schwenkbar ist,
**gekennzeichnet durch**
eine zweite Walze (120, 122, 1220a, 1220c, 1320, 1322), die an einem zweiten Griff (128, 132) angebracht ist, der zwischen den wenigstens zwei verriegelbaren Positionen mit dem ersten Griff (128, 132) schwenkbar ist, und ferner **dadurch** gekennzeichnet, dass
die Welle (104, 1204a, 1204c, 1304) **durch** die erste Walze und die zweite Walze (120, 122) läuft;
die erste Walze wenigstens einen **durch** wenigstens einen Teil der ersten Walze verlaufenden Schlitz hat;
die zweite Walze wenigstens einen **durch** wenigstens einen Teil der zweiten Walze (120, 122, 1220a, 1220c, 1320, 1322) verlaufenden Schlitz hat; und
die mehreren Wellenflügelchen (138, 1238c, 1338) und die genannten Walzenschlitze so positioniert sind, dass die genannte Welle **durch** die genannte erste und zweite Walze zwischen einer Position, in der die genannten Flügelchen durch jeweilige Schlitze der ersten und zweiten Walze vorstehen, um die erste Walze (120, 122, 1220a, 1220c, 1320, 1322) und die zweite Walze (120, 122, 1220a, 1220c, 1320, 1322) miteinander zu verriegeln, während die Welle zwischen den wenigstens zwei verriegelbaren Positionen gedreht wird, und einer anderen Position bewegbar ist, in der keines der mehreren Flügelchen in den genannten wenigstens einen Schlitz der genannten ersten Walze vorsteht und in der wenigstens eines der genannten Flügelchen **durch** wenigstens einen Schlitz der zweiten Walze vorsteht, damit sich die genannte erste Walze unabhängig von der genannten zweiten Walze drehen kann.

2. Laparoskopisches Instrument nach Anspruch 1, wobei die Welle (104, 1204a, 1204c, 1304) durch die zweite Walze (120, 122) läuft, wobei sich die zweite Walze um die Welle drehen kann, um zu bewirken, dass der zweite Griff (128, 132) zwischen den wenigstens zwei verriegelbaren Positionen schwenkbar ist.

3. Laparoskopisches Instrument nach Anspruch 1, das ferner Folgendes umfasst: eine Werkzeugwalze (118, 1318), die mit der ersten Walze und der zweiten Walze gekoppelt ist; wobei die Werkzeugwalze wenigstens einen Schlitz hat, der durch wenigstens einen Teil der Werkzeugwalze verläuft, wobei die Welle (104, 1204a, 1204c, 1304) durch die erste Walze, die zweite Walze und die Werkzeugwalze läuft, so dass sie sich zwischen den wenigstens zwei verriegelbaren Positionen drehen kann.

4. Laparoskopisches Instrument nach Anspruch 3, wobei die mehreren Flügelchen (138, 1238c, 1338) und die Walzenschlitze so positioniert sind, dass die genannte Welle durch die genannte erste Walze, die genannte zweite Walze und die genannte Werkzeugwalze zu einer Position bewegbar ist, in der die genannten Flügelchen durch jeweilige Schlitze von wenigstens zwei aus erster Walze, zweiter Walze und Werkzeugwalze vorstehen, um die genannten wenigstens zwei aus erster Walze (120, 122, 1220a, 1220c, 1320, 1322), zweiter Walze (120, 122, 1220a, 1220c, 1320, 1322) und Werkzeugwalze (118, 1318) miteinander zu verriegeln, während die Welle zwischen den wenigstens zwei verriegelbaren Positionen gedreht wird.

5. Laparoskopisches Instrument nach Anspruch 1, das ferner Folgendes umfasst: ein Gehäuse (114, 1314); die genannte Welle (104, 1204a, 1204c, 1304), die durch die erste Walze (120, 122, 1220a, 1220c, 1320, 1322) läuft, um die erste Walze entlang einer zentralen Achse des Gehäuses zu befestigen, wobei die Welle (104, 1204a, 1204c, 1304) durch die erste Walze eingesetzt wird; und einen Druckknopf, der mit der Welle verbunden ist, um die Welle zwischen einer ersten Position und einer zweiten Position zu bewegen, wobei die erste Position einer der wenigstens zwei verriegelbaren Positionen entspricht und die zweite Position einer nicht verriegelten Position entspricht.

6. Laparoskopisches Instrument nach Anspruch 5, wobei der erste Griff (128, 132) zwischen den wenigstens zwei verriegelbaren Positionen beweglich ist, wenn die Welle in der zweiten Position ist.

7. Laparoskopisches Instrument nach Anspruch 1, das ferner Folgendes umfasst: ein Werkzeug (176) mit einem Kugelende und einem Werkzeugende, wobei das Kugelende entfernbar mit einer Werkzeugwalze (118, 1318) in einer beliebigen der wenigstens zwei verriegelbaren Positionen verbunden ist.

8. Laparoskopisches Instrument nach Anspruch 7, das ferner Folgendes umfasst: die an dem Griff (128, 132) angebrachte Walze (120, 122, 1220a, 1220c, 1320, 1322), wobei die Werkzeugwalze (118, 1318) an der Walze in wenigstens einer der wenigstens zwei verriegelbaren Positionen befestigt ist.

9. Laparoskopisches Instrument nach Anspruch 1, das eine Querachse hat, entlang der eine Spitze angeordnet ist, wobei der genannte erste Griff (128, 132) ein Fixierungsgriff (132) ist, wobei die genannte erste Walze eine Fixierungswalze (122, 1322) ist, und ferner einen Auslösegriff (128) umfasst, der zwischen wenigstens zwei verriegelbaren Positionen um eine Achse orthogonal zur Querachse schwenkbar ist.

10. Laparoskopisches Instrument nach Anspruch 9, wobei die genannte zweite Walze eine an dem Auslösegriff (128) angebrachte Auslösewalze (120, 1220a, 1220c, 1320) umfasst; und ferner eine Werkzeugwalze (118, 1318) umfasst, die drehend an der Auslösewalze (128) in den wenigstens zwei verriegelbaren Positionen befestigt ist; wobei die genannte Fixierungswalze (122, 1322) an dem genannten Fixierungsgriff (132) angebracht ist, wobei die Fixierungswalze drehend an der Auslösewalze befestigt ist, wenn die Auslösewalze in einer unverriegelten Position ist.

11. Laparoskopisches Instrument nach Anspruch 10, wobei die genannte mit Flügelchen versehene Welle (104, 1204a, 1204c, 1304) dafür vorgesehen ist, die Auslösewalze (120, 1220a, 1220c, 1320), die Werkzeugwalze (118, 1318) und die Fixierungswalze (122, 1322) axial aneinander zu befestigen, wobei die Werkzeugwalze wenigstens einen Schlitz hat, der durch wenigstens einen Teil der Werkzeugwalze verläuft, wobei die mehreren Wellenflügelchen (138, 1238c, 1338) und die Schlitze so positioniert sind, dass die mit Flügelchen versehene Welle zwischen einer ersten Position und einer zweiten Position bewegbar ist, wobei die Wellenflügelchen durch jeweilige Schlitze der Werkzeugwalze (118, 1318) und der Auslösewalze (120, 1220a, 1220c, 1320) vorstehen, um die Werkzeugwalze mit Bezug auf die Auslösewalze in der ersten Position drehend zu befestigen, und die Wellenflügelchen durch jeweilige Schlitze der Auslösewalze (120, 1220a, 1220c, 1320) und der Fixierungswalze (122, 1322) vorstehen, um die Auslösewalze mit Bezug auf die Fixierungswalze in der zweiten Position drehend zu befestigen, wobei die erste Position einer der wenigstens zwei verriegelbaren Positionen entspricht.

## Revendications

1. Instrument laparoscopique, comprenant :
un arbre (104, 1204a, 1204c, 1304) incluant une pluralité d'ailettes (138, 1238c, 1338) disposées le long d'une longueur de l'arbre,
une première poignée (128, 132) apte à tourner de façon pivotante entre au moins deux positions verrouillables, et
un premier tambour (120, 122, 1220a, 1220c, 1320, 1322) attaché à la première poignée (128, 132), l'arbre passant à travers le premier tambour, et le premier tambour étant rotatif autour de l'arbre afin d'obliger la première poignée à être apte à tourner de façon pivotante entre lesdites au moins deux positions verrouillables,
**caractérisé par**
un deuxième tambour (120, 122, 1220a, 1220c, 1320, 1322), attaché à une deuxième poignée (128, 132) apte à tourner de façon pivotante entre lesdites au moins deux positions verrouillables avec la première poignée (128, 132), et en outre
**caractérisé en ce que**
l'arbre (104, 1204a, 1204c, 1304) passe à travers le premier tambour et le deuxième tambour (120, 122) ;
le premier tambour possédant au moins une fente laquelle se prolonge à travers au moins une partie du premier tambour ;
le deuxième tambour possédant au moins une fente laquelle se prolonge à travers au moins une partie du deuxième tambour (120, 122, 1220a, 1220c, 1320, 1322) ; et
la pluralité d'ailettes d'arbre (138, 1238c, 1338) et lesdites fentes de tambour sont positionnées de sorte que ledit arbre soit mobile à travers lesdits premier et deuxième tambours entre une position, dans laquelle lesdites ailettes font saillie à travers des fentes respectives des premier et deuxième tambours afin de verrouiller ensemble le premier tambour (120, 122, 1220a, 1220c, 1320, 1322) et le deuxième tambour (120, 122, 1220a, 1220c, 1320, 1322) pendant que l'arbre est tourné entre lesdites au moins deux positions verrouillables, et une autre position dans laquelle aucune ailette parmi la pluralité d'ailettes ne fait saillie dans ladite au moins une fente dudit premier tambour et dans laquelle au moins une desdites ailettes fait saillie à travers au moins une fente du deuxième tambour pour permettre audit premier tambour de tourner indépendamment par rapport audit deuxième tambour.

2. Instrument laparoscopique selon la revendication 1, l'arbre (104, 1204a, 1204c, 1304) passant à travers le deuxième tambour (120, 122), le deuxième tambour étant rotatif autour de l'arbre afin d'obliger la deuxième poignée (128, 132) à être apte à tourner de façon pivotante entre lesdites aux moins deux positions verrouillables.

3. Instrument laparoscopique selon la revendication 1, comprenant en outre : un tambour à outils (118, 1318) couplé au premier tambour et au deuxième tambour ; le tambour à outils possédant au moins une fente laquelle se prolonge à travers au moins une partie du tambour à outils, alors que l'arbre (104, 1204a, 1204c, 1304) passe à travers le premier tambour, le deuxième tambour et le tambour à outils de sorte à être rotatif entre lesdites au moins deux positions verrouillables.

4. Instrument laparoscopique selon la revendication 3, la pluralité d'ailettes (138, 1238c, 1338) et les fentes de tambour étant positionnées de sorte que ledit arbre soit mobile à travers ledit premier tambour, ledit deuxième tambour et ledit tambour à outils, vers une position dans laquelle lesdites ailettes font saillie à travers des fentes respectives d'au moins deux postes, à savoir le premier tambour, le deuxième tambour et le tambour à outils afin de verrouiller ensemble lesdits au moins deux postes, à savoir le premier tambour (120, 122, 1220a, 1220c, 1320, 1322), le deuxième tambour (120, 122, 1220a, 1220c, 1320, 1322) et le tambour à outils (118, 1318) pendant que l'arbre est tourné entre lesdites au moins deux positions verrouillables.

5. Instrument laparoscopique selon la revendication 1, comprenant en outre : un logement (114, 1314) ; ledit arbre (104, 1204a, 1204c, 1304) passant à travers le premier tambour (120, 122, 1220a, 1220c, 1320, 1322) afin d'assujettir le premier tambour le long d'un axe central du logement, l'arbre (104, 1204a, 1204c, 1304) étant inséré à travers le premier tambour ; et un bouton-poussoir connecté à l'arbre afin de déplacer l'arbre entre une première position et une deuxième position, la première position correspondant à une desdites au moins deux positions verrouillables, la deuxième position correspondant à une position déverrouillée.

6. Instrument laparoscopique selon la revendication 5, la première poignée (128, 132) étant mobile entre lesdites au moins deux positions verrouillables lorsque l'arbre se trouve dans la deuxième position.

7. Instrument laparoscopique selon la revendication 1, comprenant en outre : un outil (176) muni d'une extrémité sphérique et d'une extrémité outil, l'extrémité sphérique étant raccordée de façon détachable à un tambour à outils (118, 1318) dans l'une quelconque desdites au moins deux positions verrouillables.

8. Instrument laparoscopique selon la revendication 7, comprenant en outre le tambour (120, 122, 1220a, 1220c, 1320, 1322) attaché à la poignée (128, 132), le tambour à outils (118, 1318) étant assujetti au tambour dans au moins une desdites au moins deux positions verrouillables.

9. Instrument laparoscopique selon la revendication 1, possédant un axe transversal le long duquel est disposé un embout, cas dans lequel ladite première poignée (128, 132) est une poignée de fixation (132), cas dans lequel ledit premier tambour est un tambour de fixation (122, 1322), et comprenant en outre une poignée à déclenchement (128) apte à tourner de façon pivotante entre au moins deux positions verrouillables autour d'un axe qui est orthogonal par rapport à l'axe transversal.

10. Instrument laparoscopique selon la revendication 9, ledit deuxième tambour comprenant un tambour à déclenchement (120, 1220a, 1220c, 1320) attaché à la poignée de déclenchement (128) ; et comprenant en outre un tambour à outil (118, 1318) assujetti dans le plan rotationnel au tambour à déclenchement (128) dans lesdites au moins deux positions verrouillables ; ledit tambour de fixation (122, 1322) étant attaché à ladite poignée de fixation (132), le tambour de fixation étant assujetti dans le plan rotationnel au tambour à déclenchement lorsque le tambour à déclenchement se trouve dans une position déverrouillée.

11. Instrument laparoscopique selon la revendication 10, ledit arbre ailé (104, 1204a, 1204c, 1304) étant destiné à assujettir axialement le tambour à déclenchement (120, 1220a, 1220c, 1320), le tambour à outils (118, 1318), et le tambour de fixation (122, 1322) l'un à l'autre, le tambour à outils possédant au moins une fente laquelle se prolonge à travers au moins une partie du tambour à outils, cas dans lequel la pluralité d'ailettes d'arbre (138, 1238c, 1338) et les fentes sont positionnées de sorte que l'arbre ailé soit mobile entre une première position et une deuxième position, les ailettes d'arbre faisant saillie à travers des fentes respectives du tambour à outils (118, 1318) et du tambour à déclenchement (120, 1220a, 1220c, 1320) afin d'assujettir dans le plan rotationnel le tambour à outils par rapport au tambour de déclenchement dans la première position, et les ailettes d'arbre faisant saillie à travers des fentes respectives du tambour à déclenchement (120, 1220a, 1220c, 1320) et du tambour de fixation (122, 1322) afin d'assujettir dans le plan rotationnel le tambour de déclenchement par rapport au tambour de fixation dans la deuxième position, la première position correspondant à l'une desdites au moins deux positions verrouillables.
